(19) Europäisches Patentamt / European Patent Office / Office européen des brevets

(11) **EP 3 480 322 B1**

(12) **EUROPEAN PATENT SPECIFICATION**

(45) Date of publication and mention
of the grant of the patent:
**03.03.2021  Bulletin 2021/09**

(51) Int Cl.:
***C12Q 1/6851*** (2018.01)

(21) Application number: **18203556.8**

(22) Date of filing: **30.10.2018**

(54) **DETECTION ACCURACY IDENTIFYING METHOD, DETECTION ACCURACY IDENTIFYING DEVICE, AND DETECTION ACCURACY IDENTIFYING PROGRAM**

DETEKTIONSGENAUIGKEITIDENTIFIZIERUNGSVERFAHREN, DETEKTIONSGENAUIGKEITIDENTIFIZIERUNGSVORRICHTUNG UND DETEKTIONSGENAUIGKEITIDENTIFIZIERUNGSPROGRAMM

PROCÉDÉ D'IDENTIFICATION DE PRÉCISION DE LA DÉTECTION, DISPOSITIF D'IDENTIFICATION DE PRÉCISION DE DÉTECTION ET PROGRAMME D'IDENTIFICATION DE PRÉCISION DE DÉTECTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.11.2017  JP 2017214816**

(43) Date of publication of application:
**08.05.2019  Bulletin 2019/19**

(73) Proprietor: **Ricoh Company, Ltd.**
**Tokyo 143-8555 (JP)**

(72) Inventors:
• **SEO, Manabu**
  **Tokyo, Tokyo 143-8555 (JP)**
• **IZUMI, Satoshi**
  **Tokyo, Tokyo 143-8555 (JP)**
• **KAWASHIMA, Yudai**
  **Tokyo, Tokyo 143-8555 (JP)**

(74) Representative: **Fairbairn, Angus Chisholm et al**
**Marks & Clerk LLP**
**15 Fetter Lane**
**London EC4A 1BW (GB)**

(56) References cited:
**WO-A1-2014/043581**

• **AMIN FOROOTAN ET AL: "Methods to determine limit of detection and limit of quantification in quantitative real-time PCR (qPCR)", BIOMOLECULAR DETECTION AND QUANTIFICATION, vol. 12, 1 June 2017 (2017-06-01), pages 1-6, XP055555758, ISSN: 2214-7535, DOI: 10.1016/j.bdq.2017.04.001**
• **LIESBET DEPREZ ET AL: "Validation of a digital PCR method for quantification of DNA copy number concentrations by using a certified reference material", BIOMOLECULAR DETECTION AND QUANTIFICATION, vol. 9, 1 September 2016 (2016-09-01), pages 29-39, XP055555996, ISSN: 2214-7535, DOI: 10.1016/j.bdq.2016.08.002**
• **JUNICHI MANO ET AL: "Development of a Reference Material of a Single DNA Molecule for the Quality Control of PCR Testing", ANALYTICAL CHEMISTRY, vol. 86, no. 17, 12 August 2014 (2014-08-12), pages 8621-8627, XP055567002, US ISSN: 0003-2700, DOI: 10.1021/ac501314s**

**Description**

BACKGROUND OF THE INVENTION

Field of the Invention

The present disclosure relates to a detection accuracy identifying method, a detection accuracy identifying device, and a detection accuracy identifying program.

Description of the Related Art

In recent years, increased sensitivity of analytical techniques has enabled measurement of measurement targets in unit of the number of molecules, and industrial application of gene detection techniques for detecting trace nucleic acids to foods, environmental audits, and medical care has been demanded. Particularly, detection of pathogens or unapproved genetically modified foods is often intended for confirming absence in samples, and a detection accuracy of a high level is demanded.

For example, owing to the technological characteristics of nucleic acid detection methods utilizing nucleic acid amplification represented by polymerase chain reactions (PCR) often used in the field of molecular biology studies, the nucleic acid detection methods are said to be theoretically capable of amplifying a nucleic acid even if there is only one nucleic acid molecule.

In the detection of such trace genes by quantitative analyses, there is a need for using standard reagents, and there has been proposed a method for diluting a DNA fragment having a specific base sequence by a limiting dilution method and selecting a diluted solution including the intended number of molecules based on the result of real-time PCR of the obtained diluted solutions (for example, see Japanese Unexamined Patent Application Publication No. 2014-33658). Even in qualitative analyses for merely judging presence or absence of detection, calculation of the lower limit of detection (LOD: Limit of Detection) matters for confirmation of the validity of the test. There has been reported a method for calculating the LOD for typical tests (for example, see Chronicles of Young Scientists Vol. 2, |Issue| Jan-Mar 2011 [online], <https://cysonline.org.on Monday, June 01, 2015). However, the LOD of a nucleic acid detection method or device is of a level of some copies. Because it has been difficult to produce a stable standard substance in which the copy number is of a level of some copies, it has been difficult to measure the correct LOD.

In this regard, there has been proposed a method of introducing a specific copy number of DNA fragments into cells by a gene recombination technique, culturing the cells, and isolating the cultured cells to produce a standard reagent containing the intended copy number of DNA fragments (for example, see Japanese Unexamined Patent Application Publication No. 2015-195735). However, there has not been proposed a method for calculating an LOD employing this method.

Further, also in consideration of variation in the sample per se, there has been reported a method of repeating tests at a specific concentration and calculating a Probability of Detection (POD) for the specific concentration (see Journal of AOAC International, Volume 94, Issue 1, pp. 335-347).

Forootan et al. ("Methods to determine limit of detection and limit of quantification in quantitative real-time PCR (qPCR)", Biomolecular Detection and Quantification, 1 June 2017, vol. 12, p.1-6) discloses a method of calculating the Limit of Detection (LOD) of a qPCR system based on sample replicates, wherein the standard sample is prepared by serial dilution.

Mano et al. ("Development of a reference material of a single DNA molecule for the quality control of PCR testing", Analytical Chenistry, 12 August 2014, vol. 86(17), p.8621-8627) discloses a reference material for PCR estimated to comprise a single DNA molecule.

The present disclosure has an object to provide a detection accuracy identifying method capable of highly accurately identifying a detection accuracy ability (LOD: Limit of Detection) for detection of a low number of nucleic acid molecules.

SUMMARY OF THE INVENTION

A detection accuracy identifying method of the present disclosure is a detection accuracy identifying method for identifying a detection accuracy for detecting a testing target nucleic acid, comprising identifying a detection accuracy ability of the testing target nucleic acid, using a standard substance containing a known number of nucleic acid molecule and based on a probability at which the known number of nucleic acid molecule is detected, wherein the known number of nucleic acid molecules comprises two or more different integers.

The present disclosure can provide a detection accuracy identifying method capable of highly accurately identifying a detection accuracy ability (LOD: Limit of Detection) for detection of a low number of nucleic acid molecules.

BRIEF DESCRIPTION OF THE DRAWINGS

FIG. 1A is a diagram illustrating an example of detection results obtained by performing nucleic acid amplification and detection on known numbers (from 1 through 4) of nucleic acid molecule and calculating detection probabilities (%);
FIG. 1B is a histogram plotting a nucleic acid detection probability per number of molecules based on the results in FIG. 1A;
FIG. 2 is a histogram plotting an example of a Poisson distribution per number of nucleic acid molecules in a sample;
FIG. 3 is a curve plotting an example of a detection accuracy ability representing a non-detection probability at which nucleic acids in a sample are not detected;
FIG. 4 is a curve plotting an example of a detection accuracy ability representing a non-detection probability at which nucleic acids in a sample are not detected;
FIG. 5 is a block diagram illustrating an example of a hardware configuration of a detection accuracy identifying device;
FIG. 6 is a diagram illustrating an example of a function configuration of a detection accuracy identifying device;
FIG. 7 is a flowchart illustrating an example of a process performed according to a detection accuracy identifying program;
FIG. 8 is a perspective view illustrating an example of a testing device serving as a standard substance used in the present disclosure;
FIG. 9 is a side view illustrating an example of a testing device;
FIG. 10 is a diagram illustrating an example of positions of wells to be filled with nucleic acids in a testing device;
FIG. 11 is a diagram illustrating another example of positions of wells to be filled with nucleic acids in a testing device;
FIG. 12 is a graph plotting an example of a relationship between the frequency and the fluorescence intensity of cells in which DNA replication has occurred;
FIG. 13A is an exemplary diagram illustrating an example of an electromagnetic valve-type discharging head;
FIG. 13B is an exemplary diagram illustrating an example of a piezo-type discharging head;
FIG. 13C is an exemplary diagram illustrating a modified example of the piezo-type discharging head illustrated in FIG. 13B;
FIG. 14A is an exemplary graph plotting an example of a voltage applied to a piezoelectric element;
FIG. 14B is an exemplary graph plotting another example of a voltage applied to a piezoelectric element;
FIG. 15A is an exemplary diagram illustrating an example of a liquid droplet state;
FIG. 15B is an exemplary diagram illustrating an example of a liquid droplet state;
FIG. 15C is an exemplary diagram illustrating an example of a liquid droplet state;
FIG. 16 is a schematic diagram illustrating an example of a dispensing device configured to land liquid droplets sequentially into wells;
FIG. 17 is an exemplary diagram illustrating an example of a liquid droplet forming device;
FIG. 18 is a diagram illustrating hardware blocks of a control unit of the liquid droplet forming device of FIG. 17;
FIG. 19 is a diagram illustrating functional blocks of a control unit of the liquid droplet forming device of FIG. 17;
FIG. 20 is a flowchart illustrating an example of an operation of a liquid droplet forming device;
FIG. 21 is an exemplary diagram illustrating a modified example of a liquid droplet forming device;
FIG. 22 is an exemplary diagram illustrating another modified example of a liquid droplet forming device;
FIG. 23A is a diagram illustrating a case where two fluorescent particles are contained in a flying liquid droplet;
FIG. 23B is a diagram illustrating a case where two fluorescent particles are contained in a flying liquid droplet;
FIG. 24 is a graph plotting an example of a relationship between a luminance Li when particles do not overlap each other and a luminance Le actually measured;
FIG. 25 is an exemplary diagram illustrating another modified example of a liquid droplet forming device;
FIG. 26 is an exemplary diagram illustrating another example of a liquid droplet forming device;
FIG. 27 is an exemplary diagram illustrating an example of a method for counting cells that have passed through a micro-flow path;
FIG. 28 is an exemplary diagram illustrating an example of a method for capturing an image of a portion near a nozzle portion of a discharging head; and
FIG. 29 is a graph plotting a relationship between a probability P (>2) and an average cell number.

DESCRIPTION

(Detection accuracy identifying method, detection accuracy identifying device, and storing detection accuracy identifying program)

The detection accuracy identifying method of the present invention is a detection accuracy identifying method for identifying a detection accuracy for detecting a testing target nucleic acid, comprising identifying a detection accuracy ability of the testing target nucleic acid, using a standard substance containing a known number of nucleic acid molecule and based on a probability at which the known number of nucleic acid molecule is detected. The method may include a detection probability information obtaining step and a probability distribution obtaining step, and further includes other steps as needed.

The detection accuracy identifying device of the present invention is a detection accuracy identifying device configured to identify a detection accuracy for detecting a testing target nucleic acid, includes a detection accuracy ability identifying unit configured to identify a detection accuracy ability of the testing target nucleic acid, using a standard substance containing a known number of nucleic acid molecule and based on a probability at which the known number of nucleic acid molecule is detected, wherein the known number of nucleic acid molecules comprises two or more different integers. The device may include a detection probability information obtaining unit and a probability distribution obtaining unit, and further includes other units as needed.

The detection accuracy identifying program of the present invention is a detection accuracy identifying program for identifying a detection accuracy for detecting a testing target nucleic acid, and causes a computer to execute a process comprising identifying a detection accuracy ability of the testing target nucleic acid, using a standard substance containing a known number of nucleic acid molecule and based on a probability at which the known number of nucleic acid molecule is detected, wherein the known number of nucleic acid molecules comprises two or more different integers.

The present invention also provides a computer-readable storage medium comprising computer executable instructions that when executed by the computer will cause the computer to carry out the detection accuracy identifying method of the present invention.

Control being performed by, for example, a control unit of the detection accuracy identifying device has the same meaning as the detection accuracy identifying method being carried out. Therefore, details of the detection accuracy identifying method will also be specified through description of the detection accuracy identifying device. Further, the detection accuracy identifying program of the present invention realizes the detection accuracy identifying device of the present invention with the use of, for example, computers as hardware resources. Therefore, details of the detection accuracy identifying program of the present invention will also be specified through description of the detection accuracy identifying device of the present invention.

The detection accuracy identifying method, the detection accuracy identifying device and the detection accuracy identifying program of the present invention are based on the following finding. In the detection of nucleic acids from a sample containing a low number of nucleic acid molecules, to know the detection sensitivity, particularly, the limit of detection of the system matters for management of the accuracy of the test. According to the related art documents, a sample extracted from a sample containing a low number of nucleic acid molecules is susceptible to random variation according to a Poisson distribution depending on the amount of nucleic acids to be contained in the extracted sample. Therefore, it has been difficult to improve the accuracy of the testing device per se.

The detection accuracy identifying method, the detection accuracy identifying device and the detection accuracy identifying program of the present invention are also based on the following finding. In order to quantify a detection ability such as a limit of detection according to an existing method for calculating POD, there is a need for calculating POD at various concentrations. This needs bothersome experiments.

In the detection of testing target nucleic acid contained in a sample, even when the sample used contains a particularly low number of nucleic acid molecules, use of the detection accuracy identifying method of the present invention, the detection accuracy identifying device of the present invention, and the detection accuracy identifying program of the present invention makes it possible to identify a highly reliable detection result, i.e., a result having a high detection accuracy. Particularly, it is possible to identify a detection accuracy ability (limit of detection) highly accurately for a low number of nucleic acid molecules of 10 or less.

<Detection probability information obtaining step and detection probability information obtaining unit>

The detection probability information obtaining step is a step of obtaining information on a probability at which a nucleic acid is detected from a standard substance containing a known number of nucleic acid molecule having a specific sequence, and is performed by the detection probability information obtaining unit.

A standard substance is defined in Japanese Industrial Standards as a material or a substance having one or more characteristic values that are sufficiently uniformly and appropriately defined, in order to be used for calibration of a measuring instrument, evaluation of a measuring method, or quantification of a value of a material (JIS Q0030, ISO Guide 30). A characteristic value of the standard substance used herein is the number of nucleic acid molecules having a specific sequence. An aqueous solution containing the nucleic acid molecules is used as the standard substance.

"Known" in the term "known number of molecule" means that the number of nucleic acid molecule contained in the standard substance is in a recognizable state.

The nucleic acid contained in the standard substance is of a specific kind, and present in a known number of molecule.

The detection probability refers to a probability at which the nucleic acid contained in the standard substance and present in the known number of molecule is detected.

The detection probability information can be obtained by subjecting the nucleic acid contained in the standard substance to amplification, detecting the nucleic acid after amplification, and obtaining the rate of detection.

The "standard substance" will be described as a "testing device" including the known number of nucleic acid molecule and a containing unit configured to contain the nucleic acid. As an example of the testing device, a well plate with the known number of nucleic acid molecule will be used in the following description, the well plate being obtained by introducing the known number of nucleic acid molecule into the wells of the well plate.

The detection probability information will be described more specifically.

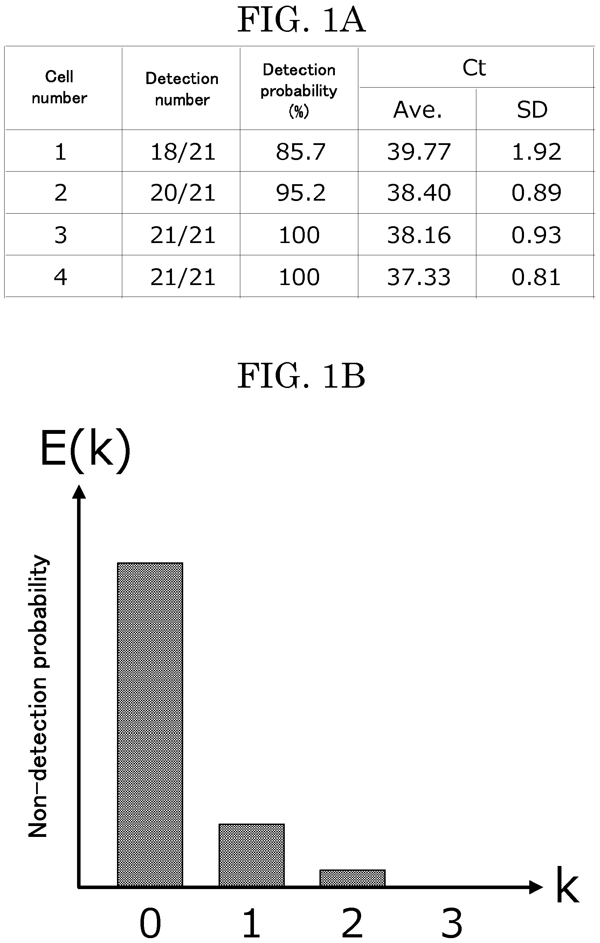

For example, an example of the standard substance, which was a testing device including from one through four cells in the wells, was subjected to amplification and detection of nucleic acids by a real-time PCR (polymerase chain reaction) method. The detection results are presented in FIG. 1A. In this example, one nucleic acid was contained in one cell. Accordingly, a relationship that the number of cells = the number of nucleic acid molecules = DNA copy number was established.

Using 21 standard substances, an experiment of detecting nucleic acids from one through four nucleic acid molecules was performed to confirm presence or absence of detection of any nucleic acid. The number of wells from which it was possible to detect nucleic acids is presented in FIG. 1A as detection number. Based on the detection number, a detection probability (%) can be calculated. In FIG. 1A, Ct (Threshold Cycle) represents a signal level at which a significant level of nucleic acid amplification by the real-time PCR method is recognized. Ave. represents an average, and SD represents standard deviation.

Next, based on the results in FIG. 1A, a non-detection probability at which the nucleic acids were not detected is plotted per number of molecules in the form of a histogram (see the results in FIG. 1B).

In the following description, k in the drawings attached to the present specification represents the number of nucleic acid molecules, and λ in the drawings represents the number of nucleic acid molecules contained in the sample.

Through such an experiment as described above, the information on the known numbers of molecule in the wells may be previously prepared in a database (DB) as information on the detection probabilities at which the nucleic acid contained in the standard substance is detected, in order that the information can be obtained from the previously prepared DB in the obtaining.

The standard substance can be used for obtaining the information on the detection probabilities.

<Probability distribution obtaining step and probability distribution obtaining unit>

The probability distribution obtaining step is a step of obtaining, in the form of a probability distribution, a variation that may occur in the detection of nucleic acids from a sample depending on the number of nucleic acid molecules contained in the sample, and is performed by the probability distribution obtaining unit.

Here, any probability distribution may be used without limitation, so long as the probability distribution can exclude an error that may occur in sampling of a sample because the sample contains a low number of molecules. Examples of the probability distribution include a Poisson distribution and a normal distribution. In the present example, it is preferable to use a Poisson distribution because a sample containing a low number of molecules is assumed.

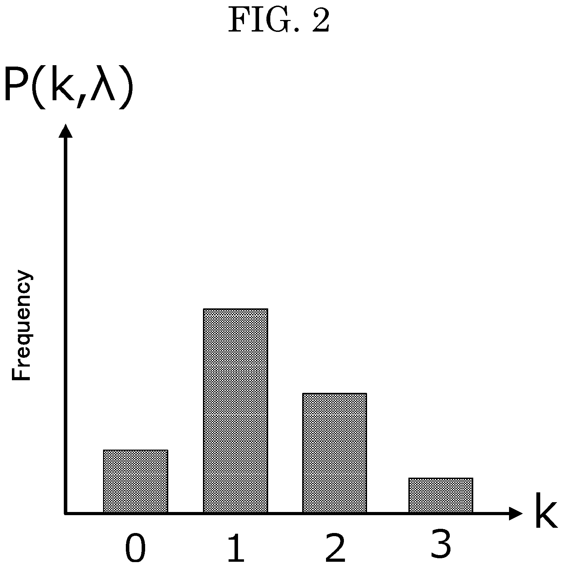

For example, an example of a histogram when the number of nucleic acid molecules in the sample is in accordance with a Poisson distribution is presented in FIG. 2.

The information on the Poisson distribution presented in FIG. 2 may be previously prepared depending on the number of nucleic acid molecules in the sample. In the probability distribution obtaining step, the previously prepared information on the Poisson distribution may be obtained in the obtaining.

<Detection accuracy ability identifying step and detection accuracy ability identifying unit>

The detection accuracy ability identifying step is a step of identifying a detection accuracy ability of a testing target nucleic acid, using a standard substance containing a known number of nucleic acid molecule and based on a probability at which the known number of nucleic acid molecule is detected, and is performed by the detection accuracy ability identifying unit.

In the detection accuracy ability identifying step, it is preferable that a detection accuracy ability of the testing target nucleic acid be identified based on a probability at which the known number of nucleic acid molecule is detected and the probability distribution.

The probability at which the known number of nucleic acid molecule is detected refers to a probability at which the known number of nucleic acid molecule contained in the standard substance is detected.

The detection probability can be obtained in the detection probability information obtaining step and by the detection probability information obtaining unit.

The probability distribution can be obtained in the probability distribution obtaining step and by the probability distribution obtaining unit.

The detection accuracy ability refers to a probability at which the testing target nucleic acid contained in a sample is detected. The detection accuracy ability represents the reliability of the result of detection of the testing target nucleic acid.

The result of detection is a pure evaluation of only the ability of a device and a reagent. Actually, the result contains an error that has occurred during sampling of the sample.

The error that has occurred during sampling of the sample is considered to conform to a random probability distribution. Therefore, the error is expressed by a Poisson distribution when the sample contains a specific number $\lambda$ of molecule (number of molecule per sample).

Accordingly, a non-detection probability E(k) with respect to a number k of molecule can be obtained by an experiment using the standard substance of the present disclosure. Here, k is a value such as 0, 1, 2, 3, ....

Next, a copy number k of DNA contained in the sample sampled in a sample amount from a population having a specific concentration $\lambda$ (number of molecule per sample) is expressed by a Poisson distribution P(k, $\lambda$) represented by a mathematical formula (1) below.

$$P(k, \lambda) = \frac{\lambda^k e^{-\lambda}}{k!}$$

···Mathematical formula (1)

Hence, when a convolution integral of E(k) with the sampling variation P(k, $\lambda$) is obtained according to a mathematical formula (2) below, a non-detection accuracy ability D($\lambda$) at the specific concentration $\lambda$ can be obtained.

$$D(\lambda) = \sum_{K=0}^{\infty} E(k) \cdot P(k, \lambda)$$

···Mathematical formula (2)

Actually, there is no case where calculation is performed from k=0 through k=infinite. In most cases, P(k, $\lambda$) becomes zero when k=5 or greater. Therefore, calculation is needed from k=0 through about k=5.

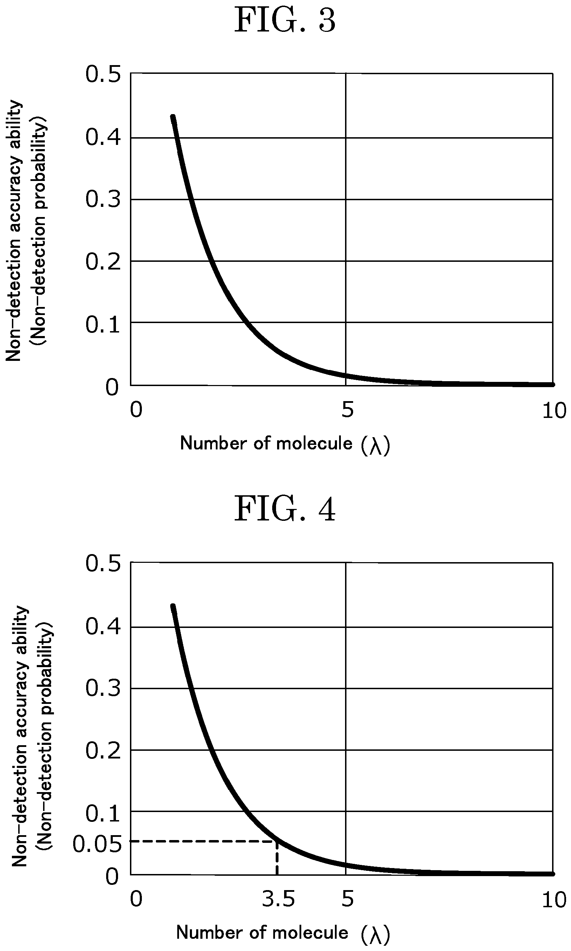

In the way described above, based on the information on the detection probability (the results in FIG. 1B) and the Poisson distribution (the results in FIG. 2), the detection probability per number of nucleic acid molecules in the sample is calculated using the mathematical formula (2). The calculation result of the nucleic acid detection probability per number of nucleic acid molecules in the sample is plotted. When the plotted points are interpolated, a detection accuracy ability curve is obtained as a function of a specific concentration (an average number of molecules in the sample) and a non-detection accuracy ability as illustrated in FIG. 3. Obtaining such a detection accuracy ability curve according to the idea of POD as in Journal of AOAC International, Volume 94, Issue 1, pp. 335-347 needs an enormous amount of procedures and has been virtually impossible. However, a detection accuracy ability curve can be obtained easily according to the method of the present disclosure.

The obtained non-detection accuracy ability (or detection accuracy ability) can be used in the manner described below in detection of nucleic acids.

Based on the result of the non-detection accuracy ability, the number of testing target molecule when the probability at which the testing target nucleic acid is present in a specific number of molecule is a predetermined value is obtained.

For example, for identification of a detection result having a detection accuracy of 95% or higher, the number of nucleic acid molecules when the non-detection probability is 0.05 is obtained as illustrated in FIG. 4. As a result, the number of nucleic acid molecules is obtained as a value of 3.5.

When the nucleic acid detection result concerned indicates the number of nucleic acid molecules as 3.5 molecules or greater, the nucleic acid detection result can be said to have a detection accuracy of 95% or higher. That is, a nucleic acid detection result having a predetermined detection accuracy can be identified. The number of nucleic acid molecules corresponding to a predetermined detection accuracy represents the limit of detection for ensuring the predetermined detection accuracy. To take the above-described case for example, any detection result that indicates a number of molecules of 3.5 or greater can be ensured a detection accuracy of 95%.

For evaluation of a nucleic acid detection accuracy, use of the detection probability (or non-detection probability) information makes it possible to evaluate the detection results by excluding errors due to the nucleic acids and a testing device.

For evaluation of a nucleic acid detection accuracy, use of a probability distribution makes it possible to evaluate the detection results by excluding errors that have occurred in sampling of the sample.

For example, from the results presented in FIG. 1A, a cell number having a detection probability of 95% or higher is obtained as 2. However, these results are obtained taking into consideration only the ability of the testing device and the reagent. Therefore, these detection results, which do not take into consideration errors that have occurred in sampling of the sample, cannot be said to be highly reliable. Moreover, there is another problem that the accuracy as an ability indicator is very coarse. For example, in a case where a detection probability when the cell number is 1 is 90% and a detection probability when the cell number 2 is 100%, a cell number having a detection probability of 95% or higher is likewise 2 according to the results presented in FIG. 1A, and the difference cannot be evaluated.

On the other hand, from the results in FIG. 4, it can be known that the number of nucleic acid molecules needed to ensure a detection accuracy ability of 95% or higher is 3.5. That is, when the number of nucleic acid molecules is 3.5 or greater, a detection result having a detection accuracy of 95% can be identified with a high reliability. For example, when the number of nucleic acid molecules is a low number, it hitherto has been unclear whether a detection result per se is a reliable one. According to the present disclosure, a result having a high reliability can be identified. Furthermore, the resolution as an ability indicator is high, and the limit of detection for the number of molecules can be calculated as a value including a decimal digit.

According to the present invention, it is possible to identify a highly reliable detection result, i.e., a result having a high detection accuracy from the results of detection from a particularly low number of nucleic acid molecules. Furthermore, it is possible to ensure the reliability of the identified result having a high detection accuracy, even when the detection result is a result of detection from a low number of nucleic acid molecules.

-Nucleic acid-

A nucleic acid means a polymeric organic compound in which a nitrogen-containing base derived from purine or pyrimidine, sugar, and phosphoric acid are bonded with one another regularly. Examples of the nucleic acid also include a fragment of a nucleic acid or an analog of a nucleic acid or of a fragment of a nucleic acid.

The nucleic acid is not particularly limited and may be appropriately selected depending on the intended purpose. Examples of the nucleic acid include DNA, RNA, and cDNA. A plasmid can also be used as the nucleic acid. The nucleic acid may be modified or mutated.

Examples of the analog of a nucleic acid or a nucleic acid fragment include a nucleic acid or a nucleic acid fragment bonded with a non-nucleic acid component, a nucleic acid or a nucleic acid fragment labeled with a labeling agent such as a fluorescent dye or an isotope (e.g., a primer or a probe labeled with a fluorescent dye or a radioisotope), and a nucleic acid or a nucleic acid fragment in which the chemical structure of some of the constituent nucleotides is changed (e.g., peptide nucleic acid). These analogs may be natural products obtained from living things, or processed products of the natural products, or products produced by utilizing a genetic recombination technique, or chemically synthesized products.

-Carrier-

It is preferable to handle the nucleic acid in a state of being carried on a carrier. The carrier is not particularly limited and may be appropriately selected depending on the intended purpose. Examples of the carrier include a cell and a resin.

The cell is not particularly limited and may be appropriately selected depending on the intended purpose so long as transgenesis can be performed in the cell. Any kinds of the cells mentioned above can be used.

It is preferable that the nucleic acid have a specific base sequence. The term "specific" means "particularly specified".

The specific base sequence is not particularly limited and may be appropriately selected depending on the intended purpose. Examples of the specific base sequence include base sequences used for infectious disease testing, naturally non-existent base sequences, animal cell-derived base sequences, and plant cell-derived base sequences. One of these base sequences may be used alone or two or more of these base sequences may be used in combination.

The nucleic acid may be a nucleic acid derived from the cells to be used, or a nucleic acid introduced by transgenesis. When a nucleic acid introduced by transgenesis and a plasmid are used as the nucleic acid, it is preferable to confirm that one copy of the nucleic acid is introduced per cell. The method for confirming that one copy of the nucleic acid is introduced is not particularly limited and may be appropriately selected depending on the intended purpose. Examples of the method include a sequencer, a PCR method, and a Southern blotting method.

The method for transgenesis is not particularly limited and may be appropriately selected depending on the intended purpose so long as the method can introduce an intended number by molecule of specific nucleic acid sequences at an intended position. Examples of the method include homologous recombination, CRISPR/Cas9, TALEN, Zinc finger nuclease, Flip-in, and Jump-in. In the case of yeast fungi, homologous recombination is preferable among these methods in terms of a high efficiency and ease of controlling.

--Cells--

A cell means a structural, functional unit that includes a nucleic acid and forms an organism.

The cells are not particularly limited and may be appropriately selected depending on the intended purpose. All kinds of cells can be used regardless of whether the cells are eukaryotic cells, prokaryotic cells, multicellular organism cells, and unicellular organism cells. One of these kinds of cells may be used alone or two or more of these kinds of cells may be used in combination.

The eukaryotic cells are not particularly limited and may be appropriately selected depending on the intended purpose. Examples of the eukaryotic cells include animal cells, insect cells, plant cells, fungi, algae, and protozoans. One of these kinds of eukaryotic cells may be used alone or two or more of these kinds of eukaryotic cells may be used in combination. Among these eukaryotic cells, animal cells and fungi are preferable.

Adherent cells may be primary cells directly taken from tissues or organs, or may be cells obtained by passaging primary cells directly taken from tissues or organs a few times. Adherent cells may be appropriately selected depending on the intended purpose. Examples of adherent cells include differentiated cells and undifferentiated cells.

Differentiated cells are not particularly limited and may be appropriately selected depending on the intended purpose. Examples of differentiated cells include: hepatocytes, which are parenchymal cells of a liver; stellate cells; Kupffer cells; endothelial cells such as vascular endothelial cells, sinusoidal endothelial cells, and corneal endothelial cells; fibroblasts; osteoblasts; osteoclasts; periodontal ligament-derived cells; epidermal cells such as epidermal keratinocytes; epithelial cells such as tracheal epithelial cells, intestinal epithelial cells, cervical epithelial cells, and corneal epithelial cells; mammary glandular cells; pericytes; muscle cells such as smooth muscle cells and myocardial cells; renal cells; pancreatic islet cells; nerve cells such as peripheral nerve cells and optic nerve cells; chondrocytes; and bone cells.

Undifferentiated cells are not particularly limited and may be appropriately selected depending on the intended purpose. Examples of undifferentiated cells include: pluripotent stem cells such as embryotic stem cells, which are undifferentiated cells, and mesenchymal stem cells having pluripotency; unipotent stem cells such as vascular endothelial progenitor cells having unipotency; and iPS cells.

Fungi are not particularly limited and may be appropriately selected depending on the intended purpose. Examples of fungi include molds and yeast fungi. One of these kinds of fungi may be used alone or two or more of these kinds of fungi may be used in combination. Among these kinds of fungi, yeast fungi are preferable because the cell cycles are adjustable and monoploids can be used.

The cell cycle means a cell proliferation process in which cells undergo cell division and cells (daughter cells) generated by the cell division become cells (mother cells) that undergo another cell division to generate new daughter cells.

Yeast fungi are not particularly limited and may be appropriately selected depending on the intended purpose. Bar1-deficient yeasts with enhanced sensitivity to a pheromone (sex hormone) that controls the cell cycle at a G1 phase are preferable. When yeast fungi are Bar1-deficient yeasts, the abundance ratio of yeast fungi with uncontrolled cell cycles can be reduced. This makes it possible to, for example, prevent a specific nucleic acid from increasing in number in the cells contained in a well.

The prokaryotic cells are not particularly limited and may be appropriately selected depending on the intended purpose. Examples of the prokaryotic cells include eubacteria and archaea. One of these kinds of prokaryotic cells may be used alone or two or more of these kinds of prokaryotic cells may be used in combination.

As the cells, dead cells are preferable. With dead cells, it is possible to prevent occurrence of cell division after fractionation.

As the cells, cells that can emit light upon reception of light are preferable. With cells that can emit light upon reception of light, it is possible to land the cells into wells while having a highly accurate control on the number of cells.

Reception of light means receiving of light.

An optical sensor means a passive sensor configured to collect, with a lens, any light in the range from visible light rays visible by human eyes to near infrared rays, short-wavelength infrared rays, and thermal infrared rays that have longer wavelengths than the visible light rays, to obtain, for example, shapes of target cells in the form of image data.

--Cells that can emit light upon reception of light --

The cells that can emit light upon reception of light are not particularly limited and may be appropriately selected depending on the intended purpose so long as the cells can emit light upon reception of light. Examples of the cells include cells stained with a fluorescent dye, cells expressing a fluorescent protein, and cells labeled with a fluorescent-labeled antibody.

A cellular site stained with a fluorescent dye, expressing a fluorescent protein, or labeled with a fluorescent-labeled antibody is not particularly limited. Examples of the cellular site include a whole cell, a cell nucleus, and a cellular membrane.

--Fluorescent dye--

Examples of the fluorescent dye include fluoresceins, azo dyes, rhodamines, coumarins, pyrenes, cyanines. One of these fluorescent dyes may be used alone or two or more of these fluorescent dyes may be used in combination. Among these fluorescent dyes, fluoresceins, azo dyes, and rhodamines are preferable, and eosin, Evans blue, trypan blue, rhodamine 6G, rhodamine B, and rhodamine 123 are more preferable.

As the fluorescent dye, a commercially available product may be used. Examples of the commercially available product include product name: EOSIN Y (available from Wako Pure Chemical Industries, Ltd.), product name: EVANS BLUE (available from Wako Pure Chemical Industries, Ltd.), product name: TRYPAN BLUE (available from Wako Pure Chemical Industries, Ltd.), product name: RHODAMINE 6G (available from Wako Pure Chemical Industries, Ltd.), product name: RHODAMINE B (available from Wako Pure Chemical Industries, Ltd.), and product name: RHODAMINE 123 (available from Wako Pure Chemical Industries, Ltd.).

--Fluorescent protein--

Examples of the fluorescent protein include Sirius, EBFP, ECFP, mTurquoise, TagCFP, AmCyan, mTFP1, MidoriishiCyan, CFP, TurboGFP, AcGFP, TagGFP, Azami-Green, ZsGreen, EmGFP, EGFP, GFP2, HyPer, TagYFP, EYFP, Venus, YFP, PhiYFP, PhiYFP-m, TurboYFP, ZsYellow, mBanana, KusabiraOrange, mOrange, TurboRFP, DsRed-Express, DsRed2, TagRFP, DsRed-Monomer, AsRed2, mStrawberry, TurboFP602, mRFP1, JRed, KillerRed, mCherry, mPlum, PS-CFP, Dendra2, Kaede, EosFP, and KikumeGR. One of these fluorescent proteins may be used alone or two or more of these fluorescent proteins may be used in combination.

--Fluorescent-labeled antibody--

The fluorescent-labeled antibody is not particularly limited and may be appropriately selected depending on the intended purpose so long as the fluorescent-labeled antibody is fluorescent-labeled. Examples of the fluorescent-labeled antibody include CD4-FITC and CD8-PE. One of these fluorescent-labeled antibodies may be used alone or two or more of these fluorescent-labeled antibodies may be used in combination.

The volume average particle diameter of the cells is preferably 100 micrometers or less, more preferably 50 micrometers or less, and particularly preferably 20 micrometers or less in a free state. When the volume average particle diameter of the cells is 100 micrometers or less, the cells can be suitably used in an inkjet method.

The volume average particle diameter of the cells can be measured by, for example, a measuring method described below.

Ten microliters is extracted from a produced stained yeast dispersion liquid and poured onto a plastic slide formed of PMMA. Then, with an automated cell counter (product name: COUNTESS AUTOMATED CELL COUNTER, available from Invitrogen), the volume average particle diameter of the cells can be measured. The cell number can be obtained by a similar measuring method.

The concentration of the cells in a cell suspension is not particularly limited, may be appropriately selected depending on the intended purpose, and is preferably $5 \times 10^4$ cells/mL or higher but $5 \times 10^8$ cells/mL or lower and more preferably $5 \times 10^4$ cells/mL or higher but $5 \times 10^7$ cells/mL or lower. When the cell number is $5 \times 10^4$ cells/mL or higher but $5 \times 10^8$ cells/mL or lower, it can be ensured that cells be contained in a discharged liquid droplet without fail. The cell number can be measured with an automated cell counter (product name: COUNTESS AUTOMATED CELL COUNTER, available from Invitrogen) in the same manner as measuring the volume average particle diameter.

The cell number of cells including a nucleic acid is not particularly limited and may be appropriately selected depending on the intended purpose so long as the cell number is a plural number.

The process of the detection accuracy identifying program of the present disclosure can be executed by a computer including a control unit that constitutes the detection accuracy identifying device.

The hardware configuration and the function configuration of the detection accuracy identifying device will be described below.

<Hardware configuration of detection accuracy identifying device>

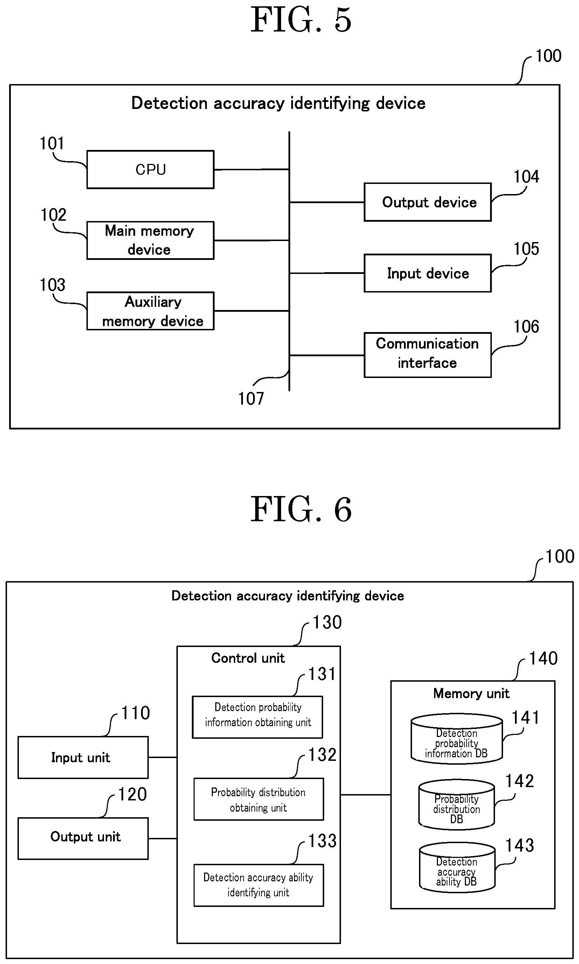

FIG. 5 is a block diagram illustrating an example of the hardware configuration of a detection accuracy identifying device 100.

As illustrated in FIG. 5, the detection accuracy identifying device 100 includes units such as a CPU (Central Processing Unit) 101, a main memory device 102, an auxiliary memory device 103, an output device 104, an input device 105, and a communication interface (communication I/F) 106. These units are coupled to one another through a bus 107.

The CPU 101 is a processing device configured to execute various controls and operations. The CPU 101 realizes various functions by executing OS (Operation System) and programs stored in, for example, the main memory device 102. That is, in the present example, the CPU 101 functions as a control unit 130 of the detection accuracy identifying device 100 by executing the detection accuracy identifying program.

The CPU 101 also controls the operation of the entire detection accuracy identifying device 100. In the present example, the CPU 101 is used as the device configured to control the operation of the entire detection accuracy identifying device 100. However, this is non-limiting. For example, FPGA (Field Programmable Gate Array) may be used.

The detection accuracy identifying program and various databases need not indispensably be stored in, for example, the main memory device 102 and the auxiliary memory device 103. The detection accuracy identifying program and various databases may be stored in, for example, another information processing device that is coupled to the detection accuracy identifying device 100 through, for example, the Internet, a LAN (Local Area Network), and a WAN (Wide Area Network). The detection accuracy identifying device 100 may receive the detection accuracy identifying program and various databases from such another information processing device and execute the program and databases.

The main memory device 102 is configured to store various programs and store, for example, data needed for execution of the various programs.

The main memory device 102 includes a ROM (Read Only Memory) and a RAM (Random Access Memory) that are not illustrated.

The ROM is configured to store various programs such as BIOS (Basic Input/Output System).

The RAM functions as a work area to be developed when the various programs stored in the ROM are executed by the CPU 101. The RAM is not particularly limited and may be appropriately selected depending on the intended purpose. Examples of the RAM include a DRAM (Dynamic Random Access Memory) and a SRAM (Static Random Access Memory).

The auxiliary memory device 103 is not particularly limited and may be appropriately selected depending on the intended purpose so long as the auxiliary memory device 103 can store various information. Examples of the auxiliary memory device 103 include portable memory devices such as a CD (Compact Disc) drive, a DVD (Digital Versatile Disc) drive, a BD (Blue-ray (registered trademark) Disc) drive.

For example, a display or a speaker can be used as the output device 104. The display is not particularly limited and a known display can be appropriately used. Examples of the display include a liquid crystal display and an organic EL display.

The input device 105 is not particularly limited and a known input device can be appropriately used so long as the input device can receive various requests to the detection accuracy identifying device 100. Examples of the input device include a keyboard, a mouse, and a touch panel.

The communication interface (communication I/F) 106 is not particularly limited and a known communication interface can be appropriately used. Examples of the communication interface include a wireless or wired communication device.

The hardware configuration as described above can realize the process functions of the detection accuracy identifying device 100.

<Function configuration of detection accuracy identifying device>

FIG. 6 is a diagram illustrating an example of the function configuration of the detection accuracy identifying device 100.

As illustrated in FIG. 6, the detection accuracy identifying device 100 includes an input unit 110, an output unit 120, the control unit 130, and a memory unit 140.

The control unit 130 includes a detection probability information obtaining unit 131, a probability distribution obtaining unit 132, and a detection accuracy ability identifying unit 133. The control unit 130 is configured to control the entire detection accuracy identifying device 100.

The memory unit 140 includes a detection probability information database 141, a probability distribution database 142, and a detection accuracy ability database 143. Hereinafter, "database" may be referred to as "DB".

The detection probability information obtaining unit 131 is configured to use detection probability information data stored in the detection probability information DB 141 of the memory unit 140 and obtain detection probability information. The detection probability information DB 141 stores, for example, data on the detection probability previously obtained through an experiment as described above. In the case of using a testing device described below, detection probability information associated with the testing device may be stored in the detection probability information DB 141. Inputs to the DB may be entered from another information processing device coupled to the detection accuracy identifying device 100 or by a human operator.

The probability distribution obtaining unit 132 is configured to use probability distribution information data stored in the probability distribution DB 142 of the memory unit 140 and obtain probability distribution information. The probability distribution DB 142 stores, for example, Poisson distribution information prepared previously.

The detection accuracy ability identifying unit 133 is configured to identify a detection accuracy ability for detecting a testing target in a sample included in a testing device, using detection probability information and a probability distribution. A specific method for identifying the detection accuracy ability is as described above.

A result calculated as the detection accuracy ability (e.g., in the example described above, the detection accuracy ability result illustrated in FIG. 3) by the detection accuracy ability identifying unit 133 is stored in the detection accuracy ability DB 143 of the memory unit 140.

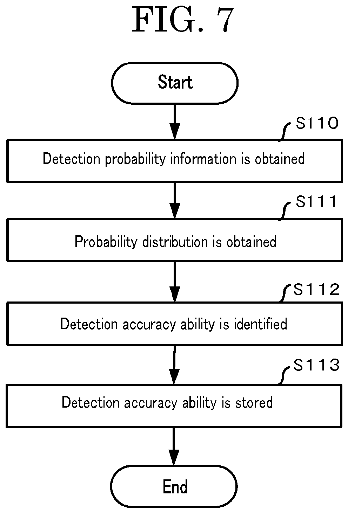

Next, the process procedures of the detection accuracy identifying program of the present disclosure will be described. FIG. 7 is a flowchart illustrating the process procedures of the detection accuracy identifying program to be performed by the control unit 130 of the detection accuracy identifying device 100.

In the step S110, the detection probability information obtaining unit 131 of the control unit 130 of the detection accuracy identifying device 100 obtains detection probability information data stored in the detection probability information DB 141 of the memory unit 140, and moves the flow to the step S111.

In the step S111, the probability distribution obtaining unit 132 of the control unit 130 of the detection accuracy identifying device 100 obtains probability distribution information data stored in the probability distribution DB 142 of the memory unit 140, and moves the flow to the step S112.

In the step S112, the detection accuracy ability identifying unit 133 of the control unit 130 of the detection accuracy identifying device 100 calculates a non-detection probability indicating the probability at which the testing target is not detected from the sample in the testing device, using detection probability information and a probability distribution, to identify the detection accuracy ability for detecting the testing target, and moves the flow to the step S113.

In the step S113, the control unit 130 of the detection accuracy identifying device 100 stores the detection accuracy ability result obtained by the detection accuracy ability identifying unit 133 in the detection accuracy ability DB 143 of the memory unit 140, and ends the process.

The testing device used with the detection accuracy identifying program of the present invention, the detection accuracy identifying method of the present invention, and the detection accuracy identifying device of the present invention will be described below.

<Testing device>

The testing device includes at least one well. In some instances, the testing device includes an identifier unit and a base material, and may further include other members as needed.

«Well»

For example, the shape, the number, the volume, the material, and the color of the well are not particularly limited and may be appropriately selected depending on the intended purpose.

The shape of the well is not particularly limited and may be appropriately selected depending on the intended purpose so long as a nucleic acid can be placed in the well. Examples of the shape of the well include: concaves such as a flat bottom, a round bottom, a U bottom, and a V bottom; and sections on a substrate.

The number of wells is at least one, and may preferably be a plural number of two or greater, more preferably five or greater, and yet more preferably 50 or greater.

It may be preferable to use a multi-well plate including two or more wells.

Examples of the multi-well plate include a 24-well, 48-well, 96-well, 384-well, or 1,536-well plate.

The volume of the well is not particularly limited, may be appropriately selected depending on the intended purpose, and may preferably be 10 microliters or greater and 1,000 microliters or less in consideration of the amount of a sample used in a common nucleic acid testing device.

The material of the well is not particularly limited and may be appropriately selected depending on the intended purpose. Examples of the material of the well include polystyrene, polypropylene, polyethylene, fluororesins, acrylic resins, polycarbonate, polyurethane, polyvinyl chloride, and polyethylene terephthalate.

Examples of the color of the well include transparent colors, semi-transparent colors, chromatic colors, and complete light-shielding colors.

«Base material»

The testing device is preferably a plate-shaped device obtained by providing a well in a base material, but may be linking-type well tubes such as 8-series tubes.

For example, the material, the shape, the size, and the structure of the base material are not particularly limited and may be appropriately selected depending on the intended purpose.

The material of the base material is not particularly limited and may be appropriately selected depending on the intended purpose. Examples of the material of the base material include semiconductors, ceramics, metals, glass, quartz glass, and plastics. Among these materials, plastics may be preferable.

Examples of the plastics include polystyrene, polypropylene, polyethylene, fluororesins, acrylic resins, polycarbonate, polyurethane, polyvinyl chloride, and polyethylene terephthalate.

The shape of the base material is not particularly limited and may be appropriately selected depending on the intended purpose. For example, board shapes and plate shapes may be preferable.

The structure of the base material is not particularly limited, may be appropriately selected depending on the intended purpose, and may be, for example, a single-layer structure or a multilayered structure.

«Identifier unit»

It may be preferable that the testing device include an identifier unit that enables detection probability information to be identified.

The identifier unit is not particularly limited and may be appropriately selected depending on the intended purpose. Examples of the identifier unit include a memory, an IC chip, a barcode, a QR code (registered trademark), a Radio Frequency Identifier (hereinafter may also be referred to as "RFID"), color coding, and printing.

The position at which the identifier unit is provided and the number of identifier units are not particularly limited and may be appropriately selected depending on the intended purpose.

Example of the information to be stored in the identifier unit include not only information indicating the number of nucleic acid molecules having a specific sequence and present in each well, but also the number of cells, the production date and time, and the name of the person in charge of production.

The information stored in the identifier unit can be read with various kinds of reading units. For example, when the identifier unit is a barcode, a barcode reader is used as the reading unit.

The method for writing information in the identifier unit is not particularly limited and may be appropriately selected depending on the intended purpose. Examples of the method include manual input, a method of directly writing data through a liquid droplet forming device configured to count the number of nucleic acids during dispensing of the nucleic acids into the wells, transfer of data stored in a server, and transfer of data stored in a cloud system.

«Other members»

The other members are not particularly limited and may be appropriately selected depending on the intended purpose. Examples of the other members include a sealing member.

-Sealing member-

In some instances, the testing device may include a sealing member in order to prevent mixing of foreign matters into the wells and outflow of the filled materials.

It may be preferable that the sealing member be configured to be capable of sealing at least one well and separable at a perforation in order to be capable of sealing or opening each one of the wells individually.

The shape of the sealing member may be a cap shape matching the inner diameter of a well, or a film shape for covering the well opening.

Examples of the material of the sealing member include polyolefin resins, polyester resins, polystyrene resins, and polyamide resins.

It may be preferable that the sealing member have a film shape that can seal all wells at a time. It may also be preferable that the sealing member be configured to have different adhesive strengths for wells that need to be reopened and wells that need not, in order that the user can reduce improper use.

Here, the known number of molecule means a copy number of nucleic acid contained in a well. As used herein, "known" in the term "known number of molecule" means that the number of nucleic acid molecule contained in each well is in a recognizable state.

The known number of molecule is preferably 10 or less and may be 5 or less.

The known number of molecules include two or more different integers.

Examples of a set of two or more different integers as known numbers of molecule include a set of 1, 2, 3, 4, 5, 6, 7, 8, 9, and 10, a set of 1, 3, 5, 7, and 9, and a set of 2, 4, 6, 8, and 10.

It may be preferable that the known number of molecule include two or more different levels, and may more preferably include a plurality of levels represented by $10^N$ (where N takes four or more continuous integers). Examples of a set of a plurality of levels include a set of following four levels: 1, 10, 100, and 1,000. This makes it possible to easily generate a calibration curve of the testing device.

It may be preferable that a well contain at least any one of a primer and an amplifying reagent.

A primer is a synthetic oligonucleotide having a complementary base sequence that includes from 18 through 30 bases and is specific to a template DNA of a polymerase chain reaction (PCR). A pair of primers, namely a forward primer and a reverse primer, are set at two positions in a manner to sandwich the region to be amplified.

Examples of the amplifying reagent for a polymerase chain reaction (PCR) include enzymes such as DNA polymerase, matrices such as the four kinds of bases (dGTP, dCTP, dATP, and dTTP), $Mg^{2+}$ (2mM magnesium chloride), and a buffer for maintaining the optimum pH (pH of from 7.5 through 9.5).

It may be preferable that the testing device include a negative control well in which the number of nucleic acid molecules is zero and a positive control well in which the number of nucleic acid molecules is 10 or greater.

Detection sensed in the negative control and non-detection sensed in the positive control suggest abnormality of the detection system (the reagent or the device). With the negative control and the positive control, the user can immediately recognize a problem when the problem occurs, and can stop the measurement and inspect the root of the problem.

Even when the known number of nucleic acid molecule having a specific sequence is 10 or less, use of this testing device makes it possible to calculate the non-detection probability of a nucleic acid detecting device, and to perform hitherto infeasible calculation of POD (Probability of Detection) or a correct LOD (limit of detection).

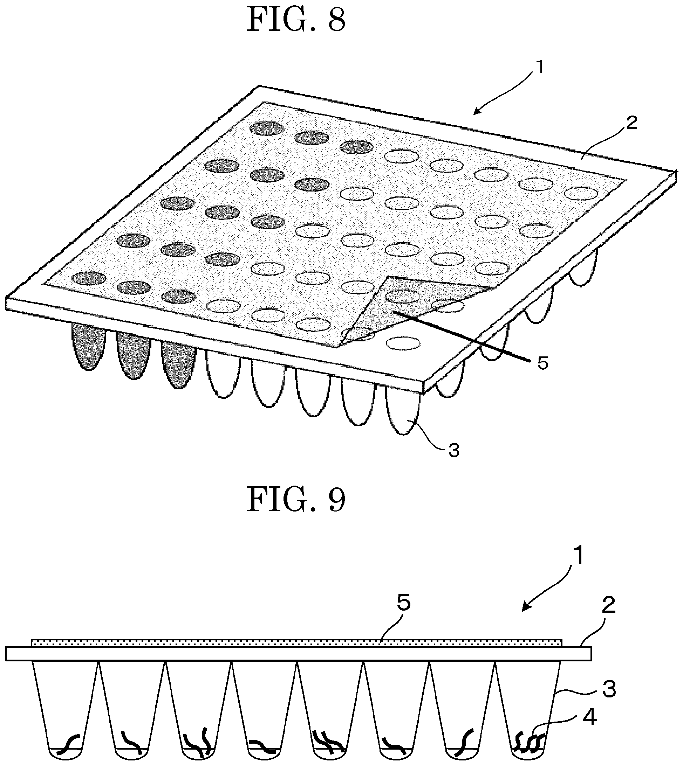

Here, FIG. 8 is a perspective view illustrating an example of a testing device 1. FIG. 9 is a side view of the testing device 1 of FIG. 8. In the testing device 1, a plurality of wells 3 are provided in a base material 2, and known numbers of nucleic acid molecule 4 having a specific sequence are filled in the wells 3. Information on the known numbers of molecule is associated with this testing device 1. The reference sign 5 in FIG. 8 and FIG. 9 denotes a sealing member.

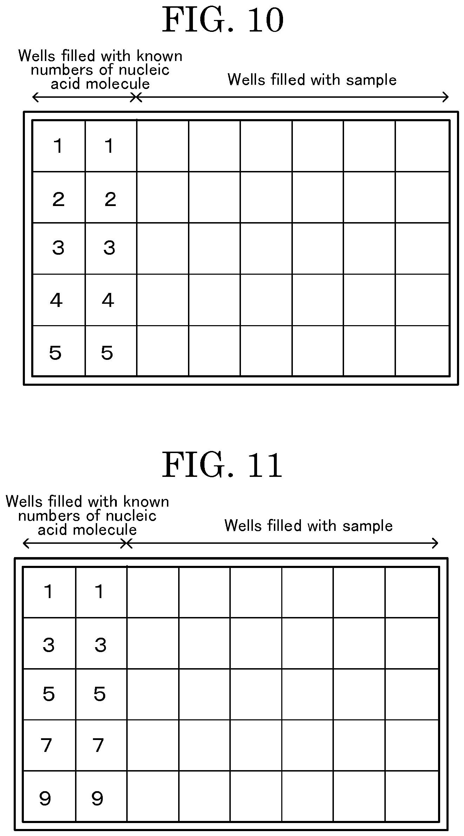

FIG. 10 is a diagram illustrating an example of the positions of the wells to be filled with nucleic acids in the testing device The numerals in the wells in FIG. 10 indicate the known numbers of nucleic acid molecule. The wells with no numerals in FIG. 10 are wells for a sample or control measurement.

FIG. 11 is a diagram illustrating another example of the positions of the wells to be filled with nucleic acids in the testing device. The numerals in the wells in FIG. 11 indicate the known numbers of nucleic acid molecule. The wells with no numerals in FIG. 11 are wells for a sample or control measurement.

<Testing device producing method>

A testing device producing method using cells containing a specific nucleic acid will be described below.

The testing device producing method may preferably include a cell suspension producing step of producing a cell suspension containing a plurality of cells including a specific nucleic acid and a solvent, a liquid droplet landing step of discharging the cell suspension in the form of liquid droplets to sequentially land the liquid droplets in wells of a plate, a cell number counting step of counting the number of cells contained in the liquid droplets with a sensor after the liquid droplets are discharged and before the liquid droplets land in the wells, a nucleic acid extracting step of extracting nucleic acids from cells in the wells, and a detection probability calculating step, and may further include other steps as needed.

«Cell suspension producing step»

The cell suspension producing step is a step of producing a cell suspension containing a plurality of cells including a specific nucleic acid and a solvent.

The solvent means a liquid used for dispersing cells.

Suspension in the cell suspension means a state of cells being present dispersedly in the solvent.

Producing means a producing operation.

-Cell suspension-

The cell suspension contains a plurality of cells including a specific nucleic acid and a solvent, preferably contains an additive, and further contains other components as needed.

The plurality of cells including a specific nucleic acid are as described above.

--Solvent--

The solvent is not particularly limited and may be appropriately selected depending on the intended purpose. Examples of the solvent include water, a culture fluid, a separation liquid, a diluent, a buffer, an organic matter dissolving liquid, an organic solvent, a polymeric gel solution, a colloid dispersion liquid, an electrolytic aqueous solution, an inorganic salt aqueous solution, a metal aqueous solution, and a mixture liquids of these liquids. One of these solvents may be used alone or two or more of these solvents may be used in combination. Among these solvents, water and a buffer are preferable, and water, a phosphate buffered saline (PBS), and a Tris-EDTA buffer (TE) are more preferable.

--Additive--

An additive is not particularly limited and may be appropriately selected depending on the intended purpose. Examples of the additive include a surfactant, a nucleic acid, and a resin. One of these additives may be used alone or two or more of these additives may be used in combination.

The surfactant can prevent mutual aggregation of cells and improve continuous discharging stability.

The surfactant is not particularly limited and may be appropriately selected depending on the intended purpose. Examples of the surfactant include ionic surfactants and nonionic surfactants. One of these surfactants may be used alone or two or more of these surfactants may be used in combination. Among these surfactants, nonionic surfactants are preferable because proteins are neither modified nor deactivated by nonionic surfactants, although depending on the addition amount of the nonionic surfactants.

Examples of the ionic surfactants include fatty acid sodium, fatty acid potassium, alpha-sulfo fatty acid ester sodium, sodium straight-chain alkyl benzene sulfonate, alkyl sulfuric acid ester sodium, alkyl ether sulfuric acid ester sodium, and sodium alpha-olefin sulfonate. One of these ionic surfactants may be used alone or two or more of these ionic surfactants may be used in combination. Among these ionic surfactants, fatty acid sodium is preferable and sodium dodecyl sulfonate (SDS) is more preferable.

Examples of the nonionic surfactants include alkyl glycoside, alkyl polyoxyethylene ether (e.g., BRIJ series), octyl phenol ethoxylate (e.g., TRITON X series, IGEPAL CA series, NONIDET P series, and NIKKOL OP series), polysorbates (e.g., TWEEN series such as TWEEN 20), sorbitan fatty acid esters, polyoxyethylene fatty acid esters, alkyl maltoside, sucrose fatty acid esters, glycoside fatty acid esters, glycerin fatty acid esters, propylene glycol fatty acid esters, and fatty acid monoglyceride. One of these nonionic surfactants may be used alone or two or more of these nonionic surfactants may be used in combination. Among these nonionic surfactants, polysorbates are preferable.

The content of the surfactant is not particularly limited, may be appropriately selected depending on the intended purpose, and is preferably 0.001% by mass or greater but 30% by mass or less relative to the total amount of the cell suspension. When the content of the surfactant is 0.001% by mass or greater, an effect of adding the surfactant can be obtained. When the content of the surfactant is 30% by mass or less, aggregation of cells can be suppressed, making it possible to strictly control the number of nucleic acid molecules in the cell suspension.

The nucleic acid is not particularly limited and may be appropriately selected depending on the intended purpose so long as the nucleic acid does not affect detection of the detection target nucleic acid. Examples of the nucleic acid include ColE1 DNA. With such a nucleic acid, it is possible to prevent the nucleic acid having a specific base sequence from adhering to the wall surface of a well.

The resin is not particularly limited and may be appropriately selected depending on the intended purpose. Examples of the resin include polyethyleneimide.

--Other materials--

Other materials are not particularly limited and may be appropriately selected depending on the intended purpose. Examples of the other materials include a cross-linking agent, a pH adjustor, an antiseptic, an antioxidant, an osmotic pressure regulator, a humectant, and a dispersant.

[Method for dispersing cells]

The method for dispersing the cells is not particularly limited and may be appropriately selected depending on the intended purpose. Examples of the method include a medium method such as a bead mill, an ultrasonic method such as an ultrasonic homogenizer, and a method using a pressure difference such as a French press. One of these methods may be used alone or two or more of these methods may be used in combination. Among these methods, the ultrasonic method is more preferable because the ultrasonic method has low damage on the cells. With the medium method, a high crushing force may destroy cellular membranes or cell walls, and the medium may mix as contamination.

[Method for screening cells]

The method for screening the cells is not particularly limited and may be appropriately selected depending on the intended purpose. Examples of the method include screening by wet classification, a cell sorter, and a filter. One of these methods may be used alone or two or more of these methods may be used in combination. Among these methods, screening by a cell sorter and a filter is preferable because the method has low damage on the cells.

It is preferable to estimate the number of nucleic acids having a specific sequence from the cell number contained in the cell suspension, by measuring the cell cycles of the cells.

Measuring the cell cycles means quantifying the cell number due to cell division.

Estimating the number of nucleic acids means estimating the copy number of nucleic acids based on the cell number.

What is to be counted needs not be the cell number, but may be the number of specific DNA sequences. Typically, it is safe to consider that the number of specific DNA sequences is equal to the cell number, because a specific DNA sequence region that is not contained per cell is selected and introduced by gene recombination. However, nucleic acid replication occurs in cells in order for the cells to undergo cell division at specific cycles. Cell cycles are different depending on the kinds of cells. By extracting a predetermined amount of the solution from the cell suspension and measuring the cycles of a plurality of cells, it is possible to calculate an expected value of the number of specific nucleic acids included in one cell and the degree of certainty of the estimated value. This can be realized by, for example, observing nuclear stained cells with a flow cytometer.

Degree of certainty means a probability of occurrence of one specific event, predicted beforehand, when there are possibilities of occurrence of some events.

Calculation means deriving a needed value by a calculating operation.

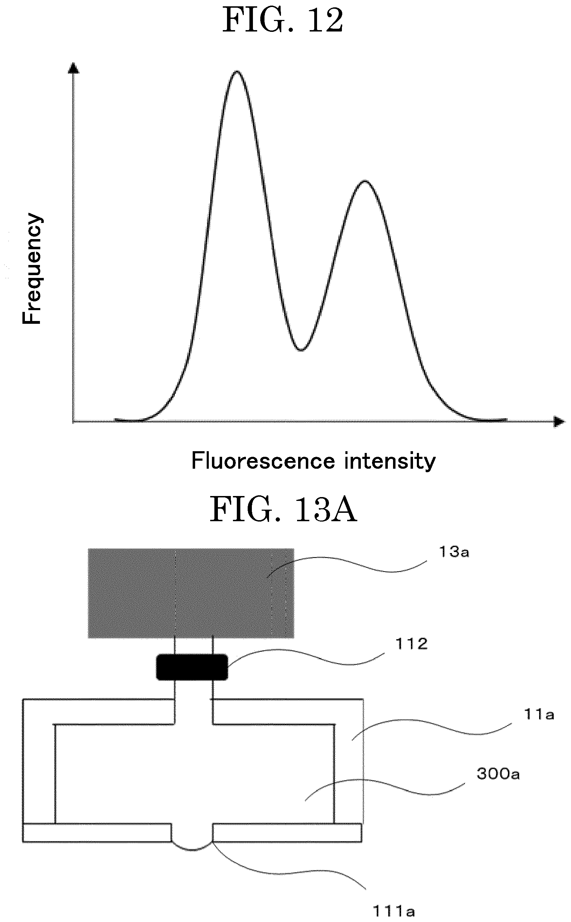

FIG. 12 is a graph plotting an example of a relationship between the frequency and the fluorescence intensity of cells in which DNA replication has occurred. As plotted in FIG. 12, based on presence or absence of DNA replication, two peaks appear on the histogram. Hence, the percentage of presence of cells in which DNA replication has occurred can be calculated. Based on this calculation result, the average DNA number included in one cell can be calculated. The estimated number of nucleic acids can be calculated by multiplying the counted cell number by the obtained average DNA number.

An operation of controlling the cell cycles before producing the cell suspension may be performed. By preparing the cells uniformly to a state before replication occurs or a state after replication has occurred, it is possible to calculate the number of specific nucleic acids based on the cell number more accurately.

A degree of certainty (probability) for the estimated number of nucleic acids may be calculated. By calculating a degree of certainty (probability), it is possible to express and output the degree of certainty as a variance or a standard deviation based on these values. When adding up influences of a plurality of factors, it is possible use a square root of the sum of the squares of the standard deviation commonly used. For example, a correct answer percentage for the number of cells discharged, the number of DNA in a cell, and a landing ratio at which discharged cells land in wells can be used as the factors. A highly influential factor may be selected for calculation.

<<Liquid droplet landing step>>

The liquid droplet landing step is a step of discharging the cell suspension in the form of liquid droplets to sequentially land the liquid droplets in wells of a plate.

A liquid droplet means a gathering of a liquid formed by a surface tension.

Discharging means making the cell suspension fly in the form of liquid droplets.

"Sequentially" means "in order".

Landing means making liquid droplets reach the wells.

As a discharging unit, a unit configured to discharge the cell suspension in the form of liquid droplets (hereinafter may also be referred to as "discharging head") can be suitably used.

Examples of the method for discharging the cell suspension in the form of liquid droplets include an on-demand method and a continuous method. Of these methods, in the case of the continuous method, there is a tendency that the dead volume of the cell suspension used is high, because of, for example, empty discharging until the discharging state becomes stable, adjustment of the amount of liquid droplets, and continued formation of liquid droplets even during transfer between the wells. In terms of cell number adjustment, it may be preferable to suppress influence due to the dead volume. Hence, of the two methods, the on-demand method may be more preferable.

Examples of the on-demand method include a plurality of known methods such as a pressure applying method of applying a pressure to a liquid to discharge the liquid, a thermal method of discharging a liquid by film boiling due to heating, and an electrostatic method of drawing liquid droplets by electrostatic attraction to form liquid droplets. Among these methods, the pressure applying method may be preferable for the reason described below.

In the electrostatic method, there is a need for disposing an electrode in a manner to face a discharging unit that is configured to retain the cell suspension and form liquid droplets. In the method for producing the device, a plate for receiving liquid droplets is disposed at the facing position. Hence, it may be preferable not to provide an electrode, in order to increase the degree of latitude in the plate configuration.

In the thermal method, there are a risk of local heating concentration that may affect the cells, which are a biomaterial, and a risk of kogation to the heater portion. Influences by heat depend on the components contained or the purpose for which the plate is used. Therefore, there is no need for flatly rejecting the thermal method. However, the pressure applying method may be preferable because the pressure applying method has a lower risk of kogation to the heater portion than the thermal method.

Examples of the pressure applying method include a method of applying a pressure to a liquid using a piezo element, and a method of applying a pressure using a valve such as an electromagnetic valve. The configuration example of a liquid droplet generating device usable for discharging liquid droplets of the cell suspension is illustrated in FIG. 13A to FIG. 13C.

FIG. 13A is an exemplary diagram illustrating an example of an electromagnetic valve-type discharging head. The electromagnetic valve-type discharging head includes an electric motor 13a, an electromagnetic valve 112, a liquid chamber 11a, a cell suspension 300a, and a nozzle 111a.

As the electromagnetic valve-type discharging head, for example, a dispenser available from Tech Elan LLC can be suitably used.

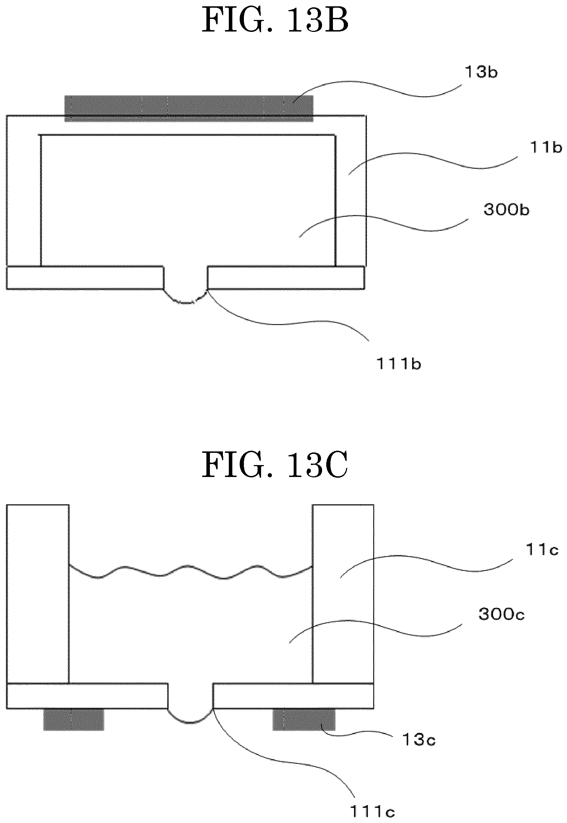

FIG. 13B is an exemplary diagram illustrating an example of a piezo-type discharging head. The piezo-type discharging head includes a piezoelectric element 13b, a liquid chamber 11b, a cell suspension 300b, and a nozzle 111b.

As the piezo-type discharging head, for example, a single cell printer available from Cytena GmbH can be suitably used.

Any of these discharging heads may be used. However, the pressure applying method by the electromagnetic valve is not capable of forming liquid droplets at a high speed repeatedly. Therefore, it may be preferable to use the piezo method in order to increase the throughput of producing a plate. A piezo-type discharging head using a common piezoelectric element 13b may cause unevenness in the cell concentration due to settlement, or may have nozzle clogging.

Therefore, a more preferable configuration may be the configuration illustrated in FIG. 13C. FIG. 13C is an exemplary diagram of a modified example of a piezo-type discharging head using the piezoelectric element illustrated in FIG. 13B. The discharging head of FIG. 13C includes a piezoelectric element 13c, a liquid chamber 11c, a cell suspension 300c, and a nozzle 111c.

In the discharging head of FIG. 13C, when a voltage is applied to the piezoelectric element 13c from an unillustrated control device, a compressive stress is applied in the horizontal direction of the drawing sheet. This can deform the membrane in the upward-downward direction of the drawing sheet.

Examples of any other method than the on-demand method include a continuous method for continuously forming liquid droplets. When pushing out liquid droplets from a nozzle by pressurization, the continuous method applies regular fluctuations using a piezoelectric element or a heater, to make it possible to continuously form minute liquid droplets. Further, the continuous method can select whether to land a flying liquid droplet into a well or to recover the liquid droplet in a recovery unit, by controlling the discharging direction of the liquid droplet with voltage application. Such a method is employed in a cell sorter or a flow cytometer. For example, a device named: CELL SORTER SH800Z available from Sony Corporation can be used.

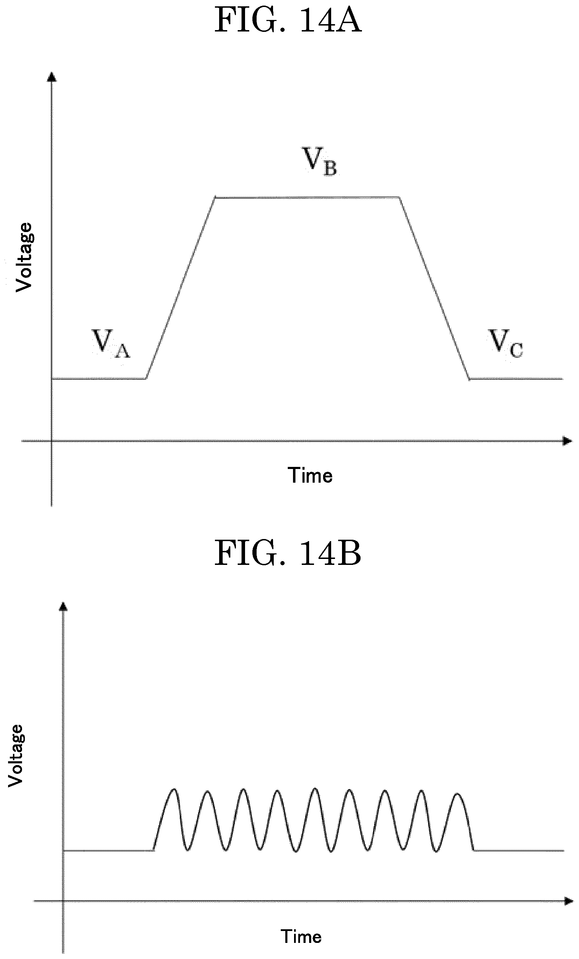

FIG. 14A is an exemplary graph plotting an example of a voltage applied to a piezoelectric element. FIG. 14B is an exemplary graph plotting another example of a voltage applied to a piezoelectric element. FIG. 14A plots a drive voltage for forming liquid droplets. Depending on the high or low level of the voltage ($V_A$, $V_B$, and $V_C$), it is possible to form liquid droplets. FIG. 14B plots a voltage for stirring the cell suspension without discharging liquid droplets.

During a period in which liquid droplets are not discharged, inputting a plurality of pulses that are not high enough to discharge liquid droplets enables the cell suspension in the liquid chamber to be stirred, making it possible to suppress occurrence of a concentration distribution due to settlement of the cells.

The liquid droplet forming operation of the discharging head that can be used in the present disclosure will be described below.

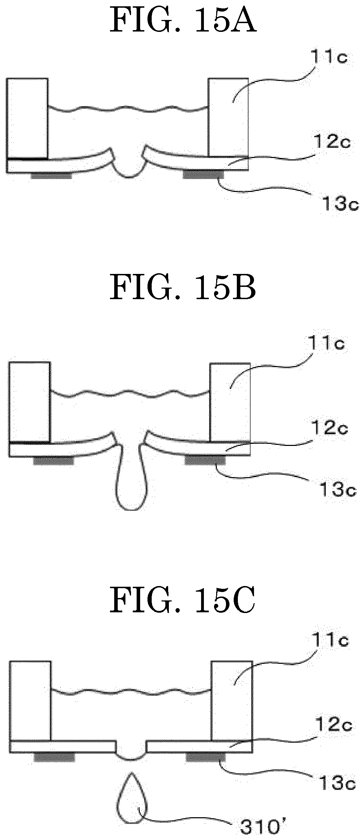

The discharging head can discharge liquid droplets with application of a pulsed voltage to the upper and lower electrodes formed on the piezoelectric element. FIG. 15A to FIG. 15C are exemplary diagrams illustrating liquid droplet states at the respective timings.

In FIG. 15A, first, upon application of a voltage to the piezoelectric element 13c, a membrane 12c abruptly deforms to cause a high pressure between the cell suspension retained in the liquid chamber 11c and the membrane 12c. This pressure pushes out a liquid droplet outward through the nozzle portion.

Next, as illustrated in FIG. 15B, for a period of time until when the pressure relaxes upward, the liquid is continuously pushed out through the nozzle portion, to grow the liquid droplet.

Finally, as illustrated in FIG. 15C, when the membrane 12c returns to the original state, the liquid pressure about the interface between the cell suspension and the membrane 12c lowers, to form a liquid droplet 310'.

In the testing device producing method, a plate in which wells are formed is secured on a movable stage, and by combination of driving of the stage with formation of liquid droplets from the discharging head, liquid droplets are sequentially landed in the concaves. A method of moving the plate along with moving the stage is described here. However, naturally, it is also possible to move the discharging head.

The plate is not particularly limited, and a plate that is commonly used in bio fields and in which wells are formed can be used.

The number of wells in the plate is not particularly limited and may be appropriately selected depending on the intended purpose. The number of wells may be a single number or a plural number.

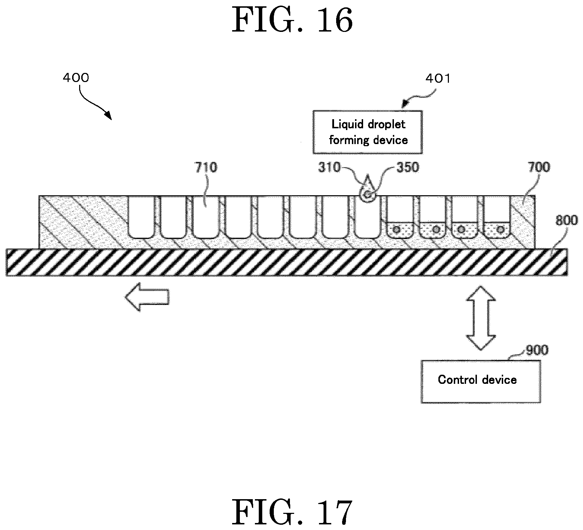

FIG. 16 is a schematic diagram illustrating an example of a dispensing device 400 configured to land liquid droplets sequentially into wells of a plate.

As illustrated in FIG. 16, the dispensing device 400 configured to land liquid droplets includes a liquid droplet forming device 401, a plate 700, a stage 800, and a control device 900.

In the dispensing device 400, the plate 700 is disposed over a movable stage 800. The plate 700 has a plurality of wells 710 (concaves) in which liquid droplets 310 discharged from a discharging head of the liquid droplet forming device 401 land. The control device 900 is configured to move the stage 800 and control the relative positional relationship between the discharging head of the liquid droplet forming device 401 and each well 710. This enables liquid droplets 310 containing fluorescent-stained cells 350 to be discharged sequentially into the wells 710 from the discharging head of the liquid droplet forming device 401.

The control device 900 may be configured to include, for example, a CPU, a ROM, a RAM, and a main memory. In this case, various functions of the control device 900 can be realized by a program recorded in, for example, the ROM being read out into the main memory and executed by the CPU. However, a part or the whole of the control device 900 may be realized only by hardware. Alternatively, the control device 900 may be configured with, for example, physically a plurality of devices.

When landing the cell suspension into the wells, it is preferable to land the liquid droplets to be discharged into the wells, in a manner that a plurality of levels are obtained.

A plurality of levels mean a plurality of references serving as standards.

As the plurality of levels, it is preferable that a plurality of cells including a specific nucleic acid have a predetermined concentration gradient in the wells. With a concentration gradient, the nucleic acid can be favorably used as a reagent for calibration curve. The plurality of levels can be controlled using values counted by a sensor.

As the plate, it may be preferable to use, for example, a 1-well microtube, 8-series tubes, a 96-well plate, and a 384-well plate. When the number of wells are a plural number, it is possible to dispense the same number of cells into the wells of these plates, or it is also possible to dispense numbers of cells of different levels into the wells. There may be a well in which no cells are contained. Particularly, for producing a plate used for evaluating a real-time PCR device or digital PCR device configured to quantitatively evaluate an amount of nucleic acids, it is preferable to dispense numbers of nucleic acids of a plurality of levels. For example, it is conceivable to produce a plate into which cells (or nucleic acids) are dispensed at 7 levels, namely about 1 cell, 2 cells, 4 cells, 8 cells, 16 cells, 32 cells, and 64 cells. Using such a plate, it is possible to inspect, for example, quantitativity, linearity, and lower limit of evaluation of a real-time PCR device or digital PCR device.

«Cell number counting step»

The cell number counting step is a step of counting the number of cells contained in the liquid droplets with a sensor after the liquid droplets are discharged and before the liquid droplets land in the wells.

A sensor means a device configured to, by utilizing some scientific principles, change mechanical, electromagnetic, thermal, acoustic, or chemical properties of natural phenomena or artificial products or spatial information/temporal information indicated by these properties into signals, which are a different medium easily handleable by humans or machines.

Counting means counting of numbers.

The cell number counting step is not particularly limited and may be appropriately selected depending on the intended purpose, so long as the cell number counting step counts the number of cells contained in the liquid droplets with a sensor after the liquid droplets are discharged and before the liquid droplets land in the wells. The cell number counting step may include an operation for observing cells before discharging and an operation for counting cells after landing.

For counting the number of cells contained in the liquid droplets after the liquid droplets are discharged and before the liquid droplets land in the wells, it may be preferable to observe cells in a liquid droplet at a timing at which the liquid droplet is at a position that is immediately above a well opening and at which the liquid droplet is predicted to enter the well in the plate without fail.

Examples of the method for observing cells in a liquid droplet include an optical detection method and an electric or magnetic detection method.

-Optical detection method-

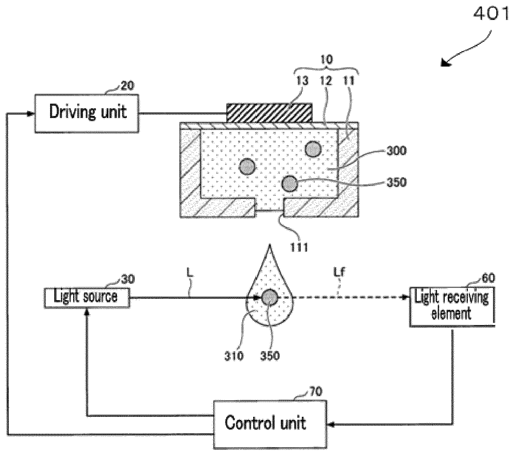

With reference to FIG. 17, FIG. 21, and FIG. 22, an optical detection method will be described below.

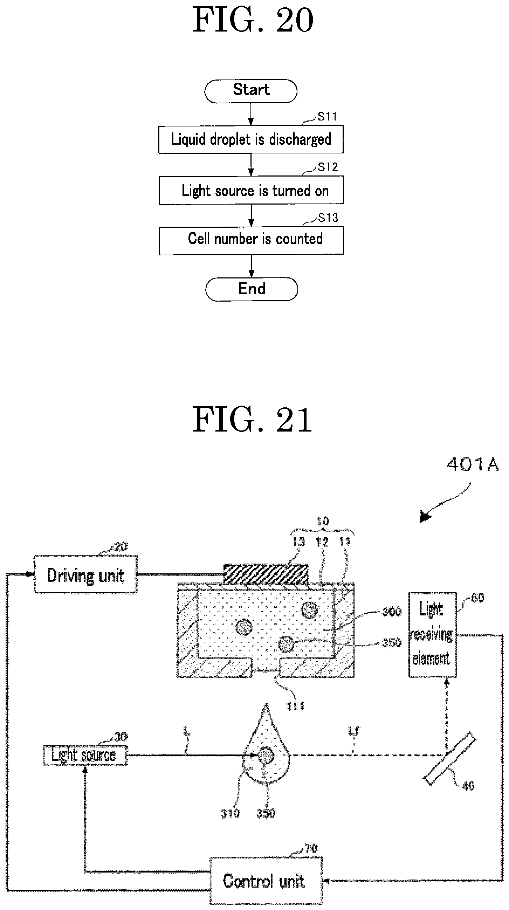

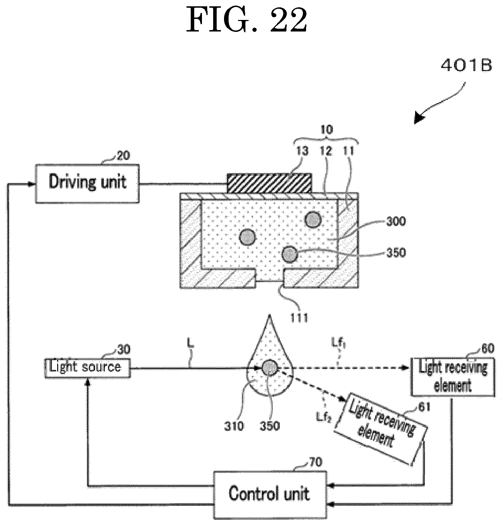

FIG. 17 is an exemplary diagram illustrating an example of a liquid droplet forming device 401. FIG. 21 and FIG. 22 are exemplary diagrams illustrating other examples of liquid droplet forming devices 401A and 401B. As illustrated in FIG. 17, the liquid droplet forming device 401 includes a discharging head (liquid droplet discharging unit) 10, a driving unit 20, a light source 30, a light receiving element 60, and a control unit 70.

In FIG. 17, a liquid obtained by dispersing cells in a predetermined solution after fluorescently staining the cells with a specific pigment is used as the cell suspension. Cells are counted by irradiating the liquid droplets formed by the discharging head with light having a specific wavelength and emitted from the light source and detecting fluorescence emitted by the cells with the light receiving element. Here, autofluorescence emitted by molecules originally contained in the cells may be utilized, in addition to the method of staining the cells with a fluorescent pigment. Alternatively, genes for producing fluorescent proteins (for example, GFP (Green Fluorescent Proteins)) may be previously introduced into the cells, in order that the cells may emit fluorescence.

Irradiation of light means application of light.

The discharging head 10 includes a liquid chamber 11, a membrane 12, and a driving element 13 and can discharge a cell suspension 300 suspending fluorescent-stained cells 350 in the form of liquid droplets.

The liquid chamber 11 is a liquid retaining portion configured to retain the cell suspension 300 suspending the fluorescent-stained cells 350. A nozzle 111, which is a through hole, is formed in the lower surface of the liquid chamber 11. The liquid chamber 11 may be formed of, for example, a metal, silicon, or a ceramic. Examples of the fluorescent-stained cells 350 include inorganic particles and organic polymer particles stained with a fluorescent pigment.

The membrane 12 is a film-shaped member secured on the upper end portion of the liquid chamber 11. The planar shape of the membrane 12 may be, for example, a circular shape, but may also be, for example, an elliptic shape or a quadrangular shape.

The driving element 13 is provided on the upper surface of the membrane 12. The shape of the driving element 13 may be designed to match the shape of the membrane 12. For example, when the planar shape of the membrane 12 is a circular shape, it may be preferable to provide a circular driving element 13.

The membrane 12 can be vibrated by supplying a driving signal to the driving element 13 from a driving unit 20. The vibration of the membrane 12 can cause a liquid droplet 310 containing the fluorescent-stained cells 350 to be discharged through the nozzle 111.

When a piezoelectric element is used as the driving element 13, for example, the driving element 13 may have a structure obtained by providing the upper surface and the lower surface of the piezoelectric material with electrodes across which a voltage is to be applied. In this case, when the driving unit 20 applies a voltage across the upper and lower electrodes of the piezoelectric element, a compressive stress is applied in the horizontal direction of the drawing sheet, making it possible for the membrane 12 to vibrate in the upward-downward direction of the drawing sheet. As the piezoelectric material, for example, lead zirconate titanate (PZT) may be used. In addition, various piezoelectric materials can be used, such as bismuth iron oxide, metal niobate, barium titanate, or materials obtained by adding metals or different oxides to these materials.

The light source 30 is configured to irradiate a flying liquid droplet 310 with light L. A flying state means a state from when the liquid droplet 310 is discharged from a liquid droplet discharging unit 10 until when the liquid droplet 310 lands on the landing target. A flying liquid droplet 310 has an approximately spherical shape at the position at which the liquid droplet 310 is irradiated with the light L. The beam shape of the light L is an approximately circular shape.

The beam diameter of the light L may be from about 10 times through 100 times as great as the diameter of the liquid droplet 310. This is for ensuring that the liquid droplet 310 is irradiated with the light L from the light source 30 without fail even when the position of the liquid droplet 310 fluctuates.

However, it may be preferable if the beam diameter of the light L is not much greater than 100 times as great as the diameter of the liquid droplet 310. This is because the energy density of the light with which the liquid droplet 310 is irradiated is reduced, to lower the light volume of fluorescence Lf to be emitted upon the light L serving as excitation light, making it difficult for the light receiving element 60 to detect the fluorescence Lf.

The light L emitted by the light source 30 may be pulse light. It may be preferable to use, for example, a solid-state laser, a semiconductor laser, and a dye laser. When the light L is pulse light, the pulse width may preferably be 10 microseconds or less and more preferably may be 1 microsecond or less. The energy per unit pulse may preferably be roughly 0.1 microjoules or higher and more preferably 1 microjoule or higher, although significantly depending on the optical system such as presence or absence of light condensation.

The light receiving element 60 is configured to receive fluorescence Lf emitted by the fluorescent-stained cell 350 upon absorption of the light L as excitation light, when the fluorescent-stained cell 350 is contained in a flying liquid droplet 310. Because the fluorescence Lf is emitted to all directions from the fluorescent-stained cell 350, the light receiving element 60 can be disposed at an arbitrary position at which the fluorescence Lf is receivable. Here, in order to improve contrast, it may be preferable to dispose the light receiving element 60 at a position at which direct incidence of the light L emitted by the light source 30 to the light receiving element 60 does not occur.

The light receiving element 60 is not particularly limited and may be appropriately selected depending on the intended purpose so long as the light receiving element 60 is an element capable of receiving the fluorescence Lf emitted by the fluorescent-stained cell 350. An optical sensor configured to receive fluorescence from a cell in a liquid droplet when the liquid droplet is irradiated with light having a specific wavelength is preferable. Examples of the light receiving element 60 include one-dimensional elements such as a photodiode and a photosensor. When high-sensitivity measurement is needed, it may be preferable to use a photomultiplier tube and an Avalanche photodiode. As the light receiving element 60, two-dimensional elements such as a CCD (Charge Coupled Device), a CMOS (Complementary Metal Oxide Semiconductor), and a gate CCD may be used.

The fluorescence Lf emitted by the fluorescent-stained cell 350 is weaker than the light L emitted by the light source 30. Therefore, a filter configured to attenuate the wavelength range of the light L may be installed at a preceding stage (light receiving surface side) of the light receiving element 60. This enables the light receiving element 60 to obtain an extremely highly contrastive image of the fluorescent-stained cell 350. As the filter, for example, a notch filter configured to attenuate a specific wavelength range including the wavelength of the light L may be used.

As described above, it may be preferable that the light L emitted by the light source 30 be pulse light. The light L emitted by the light source 30 may be continuously oscillating light. In this case, it may be preferable to control the light receiving element 60 to be capable of receiving light at a timing at which a flying liquid droplet 310 is irradiated with the continuously oscillating light, to make the light receiving element 60 receive the fluorescence Lf.

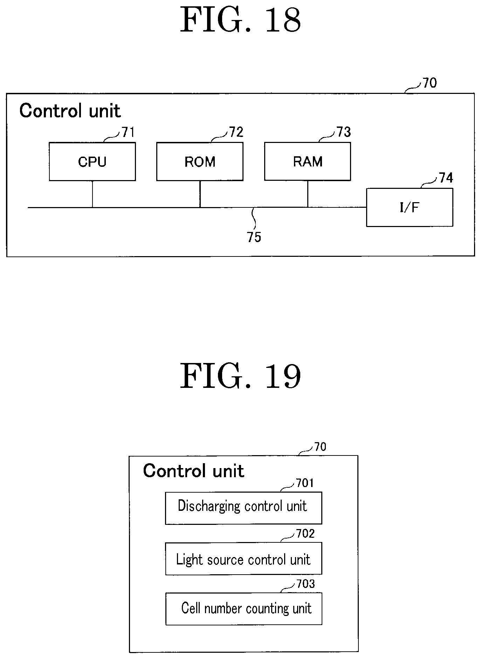

The control unit 70 has a function of controlling the driving unit 20 and the light source 30. The control unit 70 also has a function of obtaining information that is based on the light volume received by the light receiving element 60 and counting the number of fluorescent-stained cells 350 contained in the liquid droplet 310 (the case where the number is zero is also included). With reference to FIG. 18 to FIG. 20, an operation of the liquid droplet forming device 401 including an operation of the control unit 70 will be described below.

FIG. 18 is a diagram illustrating hardware blocks of the control unit of the liquid droplet forming device of FIG. 17. FIG. 19 is a diagram illustrating functional blocks of the control unit of the liquid droplet forming device of FIG. 17. FIG. 20 is a flowchart illustrating an example of the operation of the liquid droplet forming device.

As illustrated in FIG. 18, the control unit 70 includes a CPU 71, a ROM 72, a RAM 73, an I/F 74, and a bus line 75. The CPU 71, the ROM 72, the RAM 73, and the I/F 74 are coupled to one another via the bus line 75.

The CPU 71 is configured to control various functions of the control unit 70. The ROM 72 serving as a memory unit is configured to store programs to be executed by the CPU 71 for controlling the various functions of the control unit 70 and various information. The RAM 73 serving as a memory unit is configured to be used as, for example, the work area of the CPU 71. The RAM 73 is also configured to be capable of storing predetermined information for a temporary period of time. The I/F 74 is an interface configured to couple the liquid droplet forming device 401 to, for example, another device. The liquid droplet forming device 401 may be coupled to, for example, an external network via the I/F 74.

As illustrated in FIG. 19, the control unit 70 includes a discharging control unit 701, a light source control unit 702, and a cell number counting unit (cell number sensing unit) 703 as functional blocks.

With reference to FIG. 19 and FIG. 20, particle number counting by the liquid droplet forming device 401 will be described.

In the step S11, the discharging control unit 701 of the control unit 70 outputs an instruction for discharging to the driving unit 20. Upon reception of the instruction for discharging from the discharging control unit 701, the driving unit 20 supplies a driving signal to the driving element 13 to vibrate the membrane 12. The vibration of the membrane 12 causes a liquid droplet 310 containing a fluorescent-stained cell 350 to be discharged through the nozzle 111.

Next, in the step S12, the light source control unit 702 of the control unit 70 outputs an instruction for lighting to the light source 30 in synchronization with the discharging of the liquid droplet 310 (in synchronization with a driving signal supplied by the driving unit 20 to the liquid droplet discharging unit 10). In accordance with this instruction, the light source 30 is turned on to irradiate the flying liquid droplet 310 with the light L.

Here, the light is emitted by the light source 30, not in synchronization with discharging of the liquid droplet 310 by the liquid droplet discharging unit 10 (supplying of the driving signal to the liquid droplet discharging unit 10 by the driving unit 20), but in synchronization with the timing at which the liquid droplet 310 has come flying to a predetermined position in order for the liquid droplet 310 to be irradiated with the light L. That is, the light source control unit 702 controls the light source 30 to emit light at a predetermined period of time of delay from the discharging of the liquid droplet 310 by the liquid droplet discharging unit 10 (from the driving signal supplied by the driving unit 20 to the liquid droplet discharging unit 10).

For example, the speed v of the liquid droplet 310 to be discharged when the driving signal is supplied to the liquid droplet discharging unit 10 may be measured beforehand. Based on the measured speed v, the time t taken from when the liquid droplet 310 is discharged until when the liquid droplet 310 reaches the predetermined position may be calculated, in order that the timing of light irradiation by the light source 30 may be delayed from the timing at which the driving signal is supplied to the liquid droplet discharging unit 10 by the period of time of t. This enables a good control on light emission, and can ensure that the liquid droplet 310 is irradiated with the light from the light source 30 without fail.

Next, in the step S13, the cell number counting unit 703 of the control unit 70 counts the number of fluorescent-stained cells 350 contained in the liquid droplet 310 (the case where the number is zero is also included) based on information from the light receiving element 60. The information from the light receiving element 60 indicates the luminance (light volume) and the area value of the fluorescent-stained cell 350.

The cell number counting unit 703 can count the number of fluorescent-stained cells 350 by, for example, comparing the light volume received by the light receiving element 60 with a predetermined threshold. In this case, a one-dimensional element may be used or a two-dimensional element may be used as the light receiving element 60.

When a two-dimensional element is used as the light receiving element 60, the cell number counting unit 703 may use a method of performing image processing for calculating the luminance or the area of the fluorescent-stained cell 350 based on a two-dimensional image obtained from the light receiving element 60. In this case, the cell number counting unit 703 can count the number of fluorescent-stained cells 350 by calculating the luminance or the area value of the fluorescent-stained cell 350 by image processing and comparing the calculated luminance or area value with a predetermined threshold.

The fluorescent-stained cell 350 may be a cell or a stained cell. A stained cell means a cell stained with a fluorescent pigment or a cell that can express a fluorescent protein.

The fluorescent pigment for the stained cell is not particularly limited and may be appropriately selected depending on the intended purpose. Examples of the fluorescent pigment include fluoresceins, rhodamines, coumarins, pyrenes, cyanines, and azo pigments. One of these fluorescent pigments may be used alone or two or more of these fluorescent pigments may be used in combination. Among these fluorescent pigments, eosin, Evans blue, trypan blue, rhodamine 6G, rhodamine B, and Rhodamine 123 are more preferable.

Examples of the fluorescent protein include Sirius, EBFP, ECFP, mTurquoise, TagCFP, AmCyan, mTFP1, MidoriishiCyan, CFP, TurboGFP, AcGFP, TagGFP, Azami-Green, ZsGreen, EmGFP, EGFP, GFP2, HyPer, TagYFP, EYFP, Venus, YFP, PhiYFP, PhiYFP-m, TurboYFP, ZsYellow, mBanana, KusabiraOrange, mOrange, TurboRFP, DsRed-Express, DsRed2, TagRFP, DsRed-Monomer, AsRed2, mStrawberry, TurboFP602, mRFP1, JRed, KillerRed, mCherry, mPlum, PS-CFP, Dendra2, Kaede, EosFP, and KikumeGR. One of these fluorescent proteins may be used alone or two or more of these fluorescent proteins may be used in combination.

In this way, in the liquid droplet forming device 401, the driving unit 20 supplies a driving signal to the liquid droplet discharging unit 10 retaining the cell suspension 300 suspending fluorescent-stained cells 350 to cause the liquid droplet discharging unit 10 to discharge a liquid droplet 310 containing the fluorescent-stained cell 350, and the flying liquid droplet 310 is irradiated with the light L from the light source 30. Then, the fluorescent-stained cell 350 contained in the flying liquid droplet 310 emits the fluorescence Lf upon the light L serving as excitation light, and the light receiving element 60 receives the fluorescence Lf. Then, the cell number counting unit 703 counts the number of fluorescent-stained cells 350 contained in the flying liquid droplet 310, based on information from the light receiving element 60.

That is, the liquid droplet forming device 401 is configured for on-the-spot actual observation of the number of fluorescent-stained cells 350 contained in the flying liquid droplet 310. This can realize a better accuracy than hitherto obtained, in counting the number of fluorescent-stained cells 350. Moreover, because the fluorescent-stained cell 350 contained in the flying liquid droplet 310 is irradiated with the light L and emits the fluorescence Lf that is to be received by the light receiving element 60, an image of the fluorescent-stained cell 350 can be obtained with a high contrast, and the frequency of occurrence of erroneous counting of the number of fluorescent-stained cells 350 can be reduced.

FIG. 21 is an exemplary diagram illustrating a modified example of the liquid droplet forming device 401 of FIG. 17. As illustrated in FIG. 21, a liquid droplet forming device 401A is different from the liquid droplet forming device 401 (see FIG. 17) in that a mirror 40 is arranged at the preceding stage of the light receiving element 60. Description about components that are the same as in the embodiment already described may be skipped.

In the liquid droplet forming device 401A, arranging the mirror 40 at the perceiving stage of the light receiving element 60 can improve the degree of latitude in the layout of the light receiving element 60.

For example, in the layout of FIG. 17, when a nozzle 111 and a landing target are brought close to each other, there is a risk of occurrence of interference between the landing target and the optical system (particularly, the light receiving element 60) of the liquid droplet forming device 401. With the layout of FIG. 21, occurrence of interference can be avoided.

That is, by changing the layout of the light receiving element 60 as illustrated in FIG. 21, it is possible to reduce the distance (gap) between the landing target on which a liquid droplet 310 is landed and the nozzle 111 and suppress landing on a wrong position. As a result, the dispensing accuracy can be improved.

FIG. 22 is an exemplary diagram illustrating another modified example of the liquid droplet forming device 401 of FIG. 17. As illustrated in FIG. 22, a liquid droplet forming device 401B is different from the liquid droplet forming device 401 (see FIG. 17) in that a light receiving element 61 configured to receive fluorescence $Lf_2$ emitted by the fluorescent-stained cell 350 is provided in addition to the light receiving element 60 configured to receive fluorescence $Lf_1$ emitted by the fluorescent-stained cell 350. Description about components that are the same as in the embodiment already described may be skipped.

The fluorescences $Lf_1$ and $Lf_2$ represent parts of fluorescence emitted to all directions from the fluorescent-stained cell 350. The light receiving elements 60 and 61 can be disposed at arbitrary positions at which the fluorescence emitted to different directions by the fluorescent-stained cell 350 is receivable. Three or more light receiving elements may be disposed at positions at which the fluorescence emitted to different directions by the fluorescent-stained cell 350 is receivable. The light receiving elements may have the same specifications or different specifications.

With one light receiving element, when a plurality of fluorescent-stained cells 350 are contained in a flying liquid droplet 310, there is a risk that the cell number counting unit 703 may erroneously count the number of fluorescent-stained cells 350 contained in the liquid droplet 310 (a risk that a counting error may occur) because the fluorescent-stained cells 350 may overlap each other.

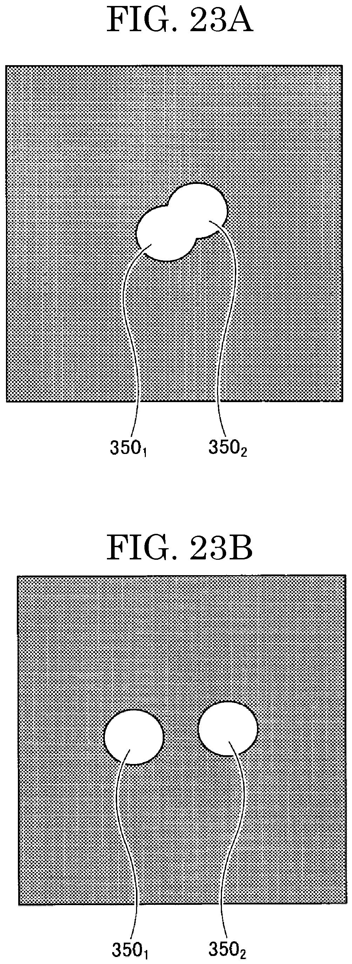

FIG. 23A and FIG. 23B are diagrams illustrating a case where two fluorescent-stained cells are contained in a flying liquid droplet. For example, as illustrated in FIG. 23A, there may be a case where fluorescent-stained cells $350_1$ and $350_2$ overlap each other, or as illustrated in FIG. 23B, there may be a case where the fluorescent-stained cells $350_1$ and $350_2$ do not overlap each other. By providing two or more light receiving elements, it is possible to reduce the influence of overlap of the fluorescent-stained cells.

As described above, the cell number counting unit 703 can count the number of fluorescent particles, by calculating the luminance or the area value of fluorescent particles by image processing and comparing the calculated luminance or area value with a predetermined threshold.

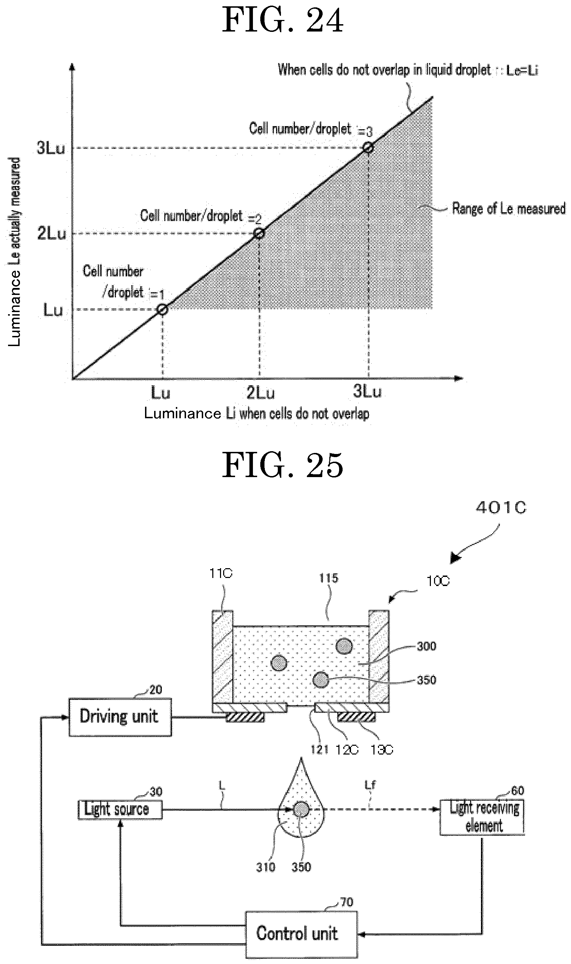

When two or more light receiving elements are installed, it is possible to suppress occurrence of a counting error, by adopting the data indicating the maximum value among the luminance values or area values obtained from these light receiving elements. This will be described in more detail with reference to FIG. 24.

FIG. 24 is a graph plotting an example of a relationship between a luminance Li when particles do not overlap each other and a luminance Le actually measured. As plotted in FIG. 24, when particles in the liquid droplet do not overlap each other, Le is equal to Li. For example, in the case where the luminance of one cell is assumed to be Lu, Le is equal to Lu when the number of cells per droplet is 1, and Le is equal to nLu when the number of particles per droplet is n (n: natural number).

However, actually, when n is 2 or greater, because particles may overlap each other, the luminance to be actually measured is $Lu \leq Le \leq nLu$ (the half-tone dot meshed portion in FIG. 24). Hence, when the number of cells per droplet is n, the threshold may be set to, for example, $(nLu-Lu/2) \leq threshold < (nLu+Lu/2)$. When a plurality of light receiving elements are installed, it is possible to suppress occurrence of a counting error, by adopting the maximum value among the data obtained from these light receiving elements. An area value may be used instead of luminance.

When a plurality of light receiving elements are installed, the number of particles may be determined according to an algorithm for estimating the number of cells based on a plurality of shape data to be obtained.

As can be understood, with the plurality of light receiving elements configured to receive fluorescence emitted to different directions by the fluorescent-stained cell 350, the liquid droplet forming device 401B can further reduce the frequency of occurrence of erroneous counting of the number of fluorescent-stained cells 350.

FIG. 25 is an exemplary diagram illustrating another modified example of the liquid droplet forming device 401 of FIG. 17. As illustrated in FIG. 25, a liquid droplet forming device 401C is different from the liquid droplet forming device 401 (see FIG. 17) in that a liquid droplet discharging unit 10C is provided instead of the liquid droplet discharging unit 10. Description about components that are the same as in the embodiment already described may be skipped.

The liquid droplet discharging unit 10C includes a liquid chamber 11C, a membrane 12C, and a driving element 13C. At the top, the liquid chamber 11C has an atmospherically exposed portion 115 configured to expose the interior of the liquid chamber 11C to the atmosphere, and bubbles mixed in the cell suspension 300 can be evacuated through the atmospherically exposed portion 115.

The membrane 12C is a film-shaped member secured at the lower end of the liquid chamber 11C. A nozzle 121, which is a through hole, is formed in approximately the center of the membrane 12C, and the vibration of the membrane 12C causes the cell suspension 300 retained in the liquid chamber 11C to be discharged through the nozzle 121 in the form of a liquid droplet 310. Because the liquid droplet 310 is formed by the inertia of the vibration of the membrane 12C, it is possible to discharge the cell suspension 300 even when the cell suspension 300 has a high surface tension (a high viscosity). The planer shape of the membrane 12C may be, for example, a circular shape, but may also be, for example, an elliptic shape or a quadrangular shape.

The material of the membrane 12C is not particularly limited. However, if the material of the membrane 12C is extremely flexible, the membrane 12C easily undergo vibration and is not easily able to stop vibration immediately when there is no need for discharging. Therefore, a material having a certain degree of hardness may be preferable. As the material of the membrane 12C, for example, a metal material, a ceramic material, and a polymeric material having a certain degree of hardness can be used.

Particularly, when a cell is used as the fluorescent-stained cell 350, the material of the membrane may preferably be a material having a low adhesiveness with the cell or proteins. Generally, adhesiveness of cells is said to be dependent on the contact angle of the material with respect to water. When the material has a high hydrophilicity or a high hydrophobicity, the material has a low adhesiveness with cells. As the material having a high hydrophilicity, various metal materials and ceramics (metal oxides) can be used. As the material having a high hydrophobicity, for example, fluororesins can be used.

Other examples of such materials include stainless steel, nickel, and aluminum, and silicon dioxide, alumina, and zirconia. In addition, it is conceivable to reduce cell adhesiveness by coating the surface of the material. For example, it is possible to coat the surface of the material with the metal or metal oxide materials described above, or coat the surface of the material with a synthetic phospholipid polymer mimicking a cellular membrane (e.g., LIPIDURE available from NOF Corporation).

It may be preferable that the nozzle 121 be formed as a through hole having a substantially perfect circle shape in approximately the center of the membrane 12C. In this case, the diameter of the nozzle 121 is not particularly limited but may preferably be twice or more greater than the size of the fluorescent-stained cell 350 in order to prevent the nozzle 121 from being clogged with the fluorescent-stained cell 350. When the fluorescent-stained cell 350 is, for example, an animal cell, particularly, a human cell, the diameter of the nozzle 121 may preferably be 10 micrometers or greater and more preferably 100 micrometers or greater in conformity with the cell used, because a human cell typically has a size of about from 5 micrometers through 50 micrometers.

On the other hand, when a liquid droplet is extremely large, it is difficult to achieve an object of forming a minute liquid droplet. Therefore, the diameter of the nozzle 121 is preferably 200 micrometers or less. That is, in the liquid droplet discharging unit 10C, the diameter of the nozzle 121 is typically in the range of from 10 micrometers through 200 micrometers.

The driving element 13C is formed on the lower surface of the membrane 12C. The shape of the driving element 13C can be designed to match the shape of the membrane 12C. For example, when the planar shape of the membrane 12C is a circular shape, it may be preferable to form a driving element 13C having an annular (ring-like) planar shape around the nozzle 121. The driving method for driving the driving element 13C may be the same as the driving method for driving the driving element 13.

The driving unit 20 can selectively (for example, alternately) apply to the driving element 13C, a discharging waveform for vibrating the membrane 12C to form a liquid droplet 310 and a stirring waveform for vibrating the membrane 12C to an extent until which a liquid droplet 310 is not formed.

For example, the discharging waveform and the stirring waveform may both be rectangular waves, and the driving voltage for the stirring waveform may be set lower than the driving voltage for the discharging waveform. This makes it possible for a liquid droplet 310 not to be formed by application of the stirring waveform. That is, it is possible to control the vibration state (degree of vibration) of the membrane 12C depending on whether the driving voltage is high or low.

In the liquid droplet discharging unit 10C, the driving element 13C is formed on the lower surface of the membrane 12C. Therefore, when the membrane 12 is vibrated by means of the driving element 13C, a flow can be generated in a direction from the lower portion to the upper portion in the liquid chamber 11C.

Here, the fluorescent-stained cells 350 move upward from lower positions, to generate a convection current in the liquid chamber 11C to stir the cell suspension 300 containing the fluorescent-stained cells 350. The flow from the lower portion to the upper portion in the liquid chamber 11C disperses the settled, aggregated fluorescent-stained cells 350 uniformly in the liquid chamber 11C.

That is, by applying the discharging waveform to the driving element 13C and controlling the vibration state of the membrane 12C, the driving unit 20 can cause the cell suspension 300 retained in the liquid chamber 11C to be discharged through the nozzle 121 in the form of a liquid droplet 310. Further, by applying the stirring waveform to the driving element 13C and controlling the vibration state of the membrane 12C, the driving unit 20 can stir the cell suspension 300 retained in the liquid chamber 11C. During stirring, no liquid droplet 310 is discharged through the nozzle 121.

In this way, stirring the cell suspension 300 while no liquid droplet 310 is being formed can prevent settlement and aggregation of the fluorescent-stained cells 350 over the membrane 12C and can disperse the fluorescent-stained cells 350 in the cell suspension 300 without unevenness. This can suppress clogging of the nozzle 121 and variation in the number of fluorescent-stained cells 350 in the liquid droplets 310 to be discharged. This makes it possible to stably discharge the cell suspension 300 containing the fluorescent-stained cells 350 in the form of liquid droplets 310 continuously for a long time.

In the liquid droplet forming device 401C, bubbles may mix in the cell suspension 300 in the liquid chamber 11C. Also in this case, with the atmospherically exposed portion 115 provided at the top of the liquid chamber 11C, the liquid droplet forming device 401C can be evacuated of the bubbles mixed in the cell suspension 300 to the outside air through the atmospherically exposed portion 115. This enables continuous, stable formation of liquid droplets 310 without a need for disposing of a large amount of the liquid for bubble evacuation.

That is, the discharging state is affected when mixed bubbles are present at a position near the nozzle 121 or when many mixed bubbles are present over the membrane 12C. Therefore, in order to perform stable formation of liquid droplets for a long time, there is a need for eliminating the mixed bubbles. Typically, mixed bubbles present over the membrane 12C move upward autonomously or by vibration of the membrane 12C. Because the liquid chamber 11C is provided with the atmospherically exposed portion 115, the mixed bubbles can be evacuated through the atmospherically exposed portion 115. This makes it possible to prevent occurrence of empty discharging even when bubbles mix in the liquid chamber 11C, enabling continuous, stable formation of liquid droplets 310.

At a timing at which a liquid droplet is not being formed, the membrane 12C may be vibrated to an extent until which a liquid droplet is not formed, in order to positively move the bubbles upward in the liquid chamber 11C.

-Electric or magnetic detection method-

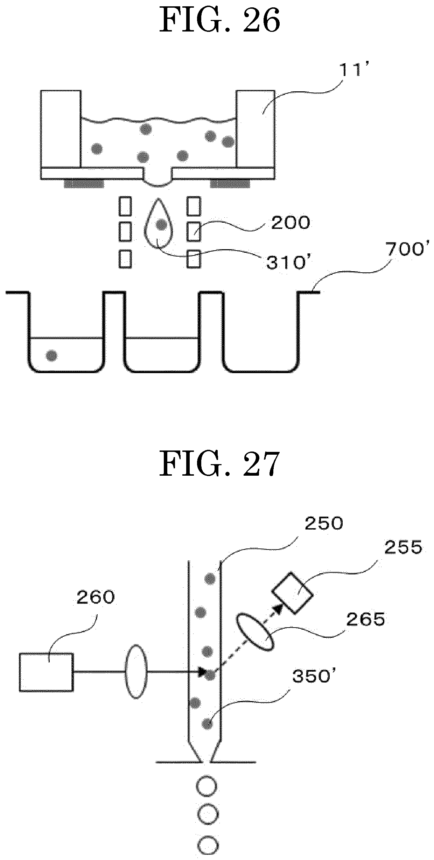

In the case of the electric or magnetic detection method, as illustrated in FIG. 26, a coil 200 configured to count the number of cells is installed as a sensor immediately below a discharging head configured to discharge the cell suspension onto a plate 700' from a liquid chamber 11' in the form of a liquid droplet 310'. Cells are modified with a specific protein and coated with magnetic beads that can adhere to the cells. Therefore, when the cells to which magnetic beads adhere pass through the coil, an induced current is generated to enable detection of presence or absence of the cells in the flying liquid droplet. Generally, cells have proteins specific to the cells on the surfaces of the cells. Modification of magnetic beads with antibodies that can adhere to the proteins enables adhesion of the magnetic beads to the cells. As such magnetic beads, a ready-made product can be used. For example, DYNABEADS (registered trademark) available from Veritas Corporation can be used.

[Operation for observing cells before discharging]

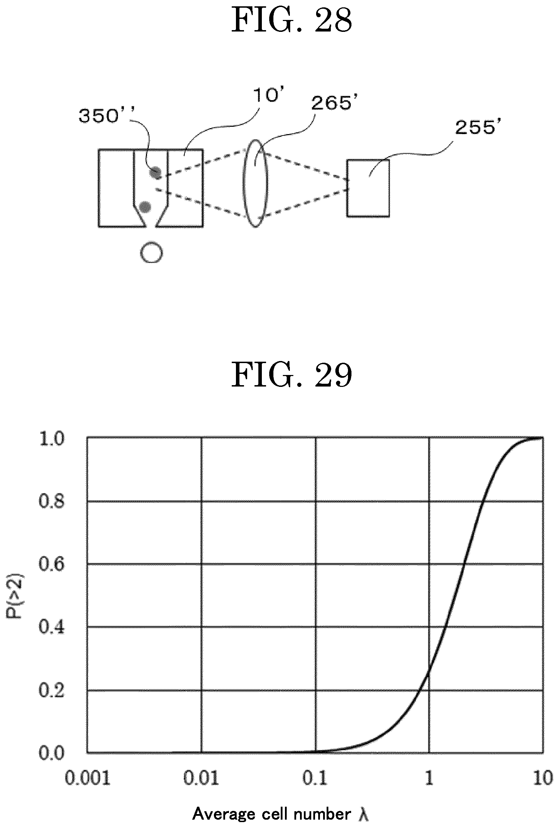

The operation for observing cells before discharging may be performed by, for example, a method for counting cells 350' that have passed through a micro-flow path 250 illustrated in FIG. 27 or a method for capturing an image of a portion near a nozzle portion of a discharging head illustrated in FIG. 28. The method of FIG. 27 is a method used in a cell sorter device, and, for example, CELL SORTER SH800Z available from Sony Corporation can be used. In FIG. 27, a light source 260 emits laser light into the micro-flow path 250, and a detector 255 detects scattered light or fluorescence through a condenser lens 265. This enables discrimination of presence or absence of cells or the kind of the cells, while a liquid droplet is being formed. Based on the number of cells that have passed through the micro-flow path 250, this method enables estimation of the number of cells that have landed in a predetermined well.

As the discharging head 10' illustrated in FIG. 28, a single cell printer available from Cytena GmbH can be used. In FIG. 28, it is possible to estimate the number of cells that have landed in a predetermined well, by capturing an image of the portion near the nozzle portion with an image capturing unit 255' through a lens 265' before discharging and estimating based on the captured image that cells 350" present near the nozzle portion have been discharged, or by estimating the number of cells that are considered to have been discharged based on a difference between images captured before and after discharging. The method of FIG. 28 is more preferable because the method enables on-demand liquid droplet formation, whereas the method of FIG. 27 for counting cells that have passed through the micro-flow path generates liquid droplets continuously.

[Operation for counting cells after landing]

The operation for counting cells after landing may be performed by a method for detecting fluorescent-stained cells by observing the wells in the plate with, for example, a fluorescence microscope. This method is described in, for example, Sangjun et al., PLoS One, Volume 6(3), e17455.

Methods for observing cells before discharging a liquid droplet or after landing have the problems described below. Depending on the kind of the plate to be produced, it is the most preferable to observe cells in a liquid droplet that is being discharged. In the method for observing cells before discharging, the number of cells that are considered to have landed is counted based on the number of cells that have passed through a flow path and image observation before discharging (and after discharging). Therefore, it is not confirmed whether the cells have actually been discharged, and an unexpected error may occur. For example, there may be a case where because the nozzle portion is stained, a liquid droplet is not discharged appropriately but adheres to the nozzle plate, thus failing to make the cells in the liquid droplet land. Moreover, there may occur a problem that the cells stay behind in a narrow region of the nozzle portion, or a discharging operation causes the cells to move beyond assumption and go outside the range of observation.

The method for detecting cells on the plate after landing also have problems. First, there is a need for preparing a plate that can be observed with a microscope. As a plate that can be observed, it is common to use a plate having a transparent, flat bottom surface, particularly a plate having a bottom surface formed of glass. However, there is a problem that such a special plate is incompatible with use of ordinary wells. Further, when the number of cells is large, such as some tens of cells, there is a problem that correct counting is impossible because the cells may overlap with each other. Accordingly, it may be preferable to perform the operation for observing cells before discharging and the operation for counting cells after landing, in addition to counting the number of cells contained in a liquid droplet with a sensor and a particle number (cell number) counting unit after the liquid droplet is discharged and before the liquid droplet lands in a well.

As the light receiving element, a light receiving element including one or a small number of light receiving portion(s), such as a photodiode, an Avalanche photodiode, and a photomultiplier tube may be used. In addition, a two-dimensional sensor including light receiving elements in a two-dimensional array formation, such as a CCD (Charge Coupled Device), a CMOS (Complementary Metal Oxide Semiconductor), and a gate CCD may be used.

When using a light receiving element including one or a small number of light receiving portion(s), it is conceivable to determine the number of cells contained, based on the fluorescence intensity, using a calibration curve prepared beforehand. Here, binary detection of whether cells are present or absent in a flying liquid droplet is common. When the cell suspension is discharged in a state that the cell concentration is so sufficiently low that almost only 1 or 0 cell(s) will be contained in a liquid droplet, sufficiently accurate counting is available by the binary detection. On the premise that cells are randomly distributed in the cell suspension, the cell number in a flying liquid droplet is considered to conform to a Poisson distribution, and the probability P (>2) at which two or more cells are contained in a liquid droplet is represented by a formula (1) below. FIG. 29 is a graph plotting a relationship between the probability P (>2) and an average cell number. Here, $\lambda$ is a value representing an average cell number in a liquid droplet and obtained by multiplying the cell concentration in the cell suspension by the volume of a liquid droplet discharged.

$$P(>2) = 1-(1+\lambda)\times e^{-\lambda} \cdots \text{formula (1)}$$

When performing cell number counting by binary detection, in order to ensure accuracy, it may be preferable that the probability P (>2) be a sufficiently low value, and that $\lambda$ satisfy: $\lambda<0.15$, at which the probability P (>2) is 1% or lower. The light source is not particularly limited and may be appropriately selected depending on the intended purpose, so long as the light source can excite fluorescence from cells. It is possible to use, for example, an ordinary lamp such as a mercury lamp and a halogen lamp to which a filter is applied for emission of a specific wavelength, a LED (Light Emitting Diode), and a laser. However, particularly when forming a minute liquid droplet of 1 nL or less, there is a need for irradiating a small region with a high light intensity. Therefore, use of a laser may be preferable. As a laser light source, various commonly known lasers such as a solid-state laser, a gas laser, and a semiconductor laser can be used. The excitation light source may be a light source that is configured to continuously irradiate a region through which a liquid droplet passes or may be a light source that is configured for pulsed irradiation in synchronization with discharging of a liquid droplet at a timing delayed by a predetermined period of time from the operation for discharging the liquid droplet.

<<Step of calculating degrees of certainty of estimated numbers of nucleic acids in cell suspension producing step, liquid droplet landing step, and cell number counting step>>

The step of calculating degrees of certainty of estimated numbers of nucleic acids in the cell suspension producing step, the liquid droplet landing step, and the cell number counting step is a step of calculating the degree of certainty in each of the cell suspension producing step, the liquid droplet landing step, and the cell number counting step.

The degree of certainty of an estimated number of nucleic acids can be calculated in the same manner as calculating the degree of certainty in the cell suspension producing step.

The timing at which the degrees of certainty are calculated may be collectively in the next step to the cell number counting step, or may be at the end of each of the cell suspension producing step, the liquid droplet landing step, and the cell number counting step in order for the degrees of certainty to be summed in the next step to the cell number counting step. In other words, the degrees of certainty in these steps need only to be calculated at arbitrary timings by the time when summing is performed.

<<Outputting step>>

The outputting step is a step of outputting a counted value of the number of cells contained in the cell suspension that has landed in a well, counted by a particle number counting unit based on a detection result measured by a sensor.

The counted value means a number of cells contained in the well, calculated by the particle number counting unit based on the detection result measured by the sensor.

Outputting means sending a value counted by a device such as a motor, communication equipment, and a calculator upon reception of an input to an external server serving as a count result memory unit in the form of electronic information, or printing the counted value as a printed matter.

In the outputting step, an observed value or an estimated value obtained by observing or estimating the number of cells or the number of nucleic acids in each well of a plate during production of the plate is output to an external memory unit.

Outputting may be performed at the same time as the cell number counting step, or may be performed after the cell number counting step.

<<Recording step>>

The recording step is a step of recording the observed value or the estimated value output in the outputting step.

The recording step can be suitably performed by a recording unit.

Recording may be performed at the same time as the outputting step, or may be performed after the outputting step.

Recording means not only supplying information to a recording medium but also storing information in a memory unit.

<<Nucleic acid extracting step>>

The nucleic acid extracting step is a step of extracting nucleic acids from cells in the well.

Extracting means destroying, for example, cellular membranes and cell walls to pick out nucleic acids.

As the method for extracting nucleic acids from cells, there is known a method of thermally treating cells at from 90 degrees C through 100 degrees C. By a thermal treatment at 90 degrees C or lower, there is a possibility that DNA may not be extracted. By a thermal treatment at 100 degrees C or higher, there is a possibility that DNA may be decomposed. Here, it may be preferable to perform thermal treatment with addition of a surfactant.

The surfactant is not particularly limited and may be appropriately selected depending on the intended purpose. Examples of the surfactant include ionic surfactants and nonionic surfactants. One of these surfactants may be used alone or two or more of these surfactants may be used in combination. Among these surfactants, nonionic surfactants may be preferable because proteins are neither modified nor deactivated by nonionic surfactants, although depending on the addition amount of the nonionic surfactants.

Examples of the ionic surfactants include fatty acid sodium, fatty acid potassium, alpha-sulfo fatty acid ester sodium, sodium straight-chain alkyl benzene sulfonate, alkyl sulfuric acid ester sodium, alkyl ether sulfuric acid ester sodium, and sodium alpha-olefin sulfonate. One of these ionic surfactants may be used alone or two or more of these ionic surfactants may be used in combination. Among these ionic surfactants, fatty acid sodium is preferable and sodium dodecyl sulfate (SDS) is more preferable.

Examples of the nonionic surfactants include alkyl glycoside, alkyl polyoxyethylene ether (e.g., BRIJ series), octyl phenol ethoxylate (e.g., TRITON X series, IGEPAL CA series, NONIDET P series, and NIKKOL OP series), polysorbates (e.g., TWEEN series such as TWEEN 20), sorbitan fatty acid esters, polyoxyethylene fatty acid esters, alkyl maltoside, sucrose fatty acid esters, glycoside fatty acid esters, glycerin fatty acid esters, propylene glycol fatty acid esters, and fatty acid monoglyceride. One of these nonionic surfactants may be used alone or two or more of these nonionic surfactants may be used in combination. Among these nonionic surfactants, polysorbates may be preferable.

The content of the surfactant is preferably 0.01% by mass or greater but 5.00% by mass or less relative to the total amount of the cell suspension in the well. When the content of the surfactant is 0.01% by mass or greater, the surfactant can be effective for DNA extraction. When the content of the surfactant is 5.00% by mass or less, inhibition against amplification can be prevented during PCR. As a numerical range in which both of these effects can be obtained, the range of 0.01% by mass or greater but 5.00% by mass or less may be preferable.

The method described above may not be able to sufficiently extract DNA from a cell that has a cell wall. Examples of methods for such a case include an osmotic shock procedure, a freeze-thaw method, an enzymic digestive method, use of a DNA extraction kit, an ultrasonic treatment method, a French press method, and a homogenizer method. Among these methods, an enzymic digestive method may be preferable because the method can save loss of extracted DNA.

<<Detection probability calculating step>>

For example, when a well plate is used as a standard substance, detection probability information can be obtained by amplifying nucleic acids contained in a well, detecting the number of nucleic acid molecules after the amplification, and comparing the number of molecules obtained in the experiment with a known number of molecule.

When a nucleic acid contained in the well can be detected in the experiment, it is judged that a nucleic acid is present in the well of the well plate. On the other hand, when no nucleic acid can be detected, it is judged that a nucleic acids is absent (not detected) in the well of the well plate.

Specifically, the detection probability can be calculated in the manner described below.

One through four cells contained in the wells of the well plate were subjected to amplification and detection of nucleic acids by a real-time PCR (polymerase chain reaction) method. The detection results are presented in FIG. 1A. In this Example, one nucleic acid was contained in one cell. Accordingly, a relationship that the number of cells = the number of nucleic acid molecules = DNA copy number was established.

Using 21 well plates including nucleic acids, an experiment of detecting nucleic acids from one through four nucleic acid molecules was performed to confirm presence or absence of a nucleic acid. The detection number is presented in FIG. 1A. Based on the detection number, a detection probability (%) can be calculated.

<<Other steps>>

The other steps are not particularly limited and may be appropriately selected depending on the intended purpose. Examples of the other steps include an enzyme deactivating step.

-Enzyme deactivating step-

The enzyme deactivating step is a step of deactivating an enzyme.

Examples of the enzyme include DNase, RNase, and an enzyme used in the nucleic acid extracting step in order to extract a nucleic acid.

The method for deactivating an enzyme is not particularly limited and may be appropriately selected depending on the intended purpose. A known method can be suitably used.

The testing device of the present disclosure is widely used in, for example, biotechnology-related industries, life science industries, and health care industries, and can be used suitably for, for example, equipment calibration or generation of calibration curves, and management of the accuracy of a testing device.

In the case of working the testing device for infectious diseases, the testing device is applicable to methods stipulated as official analytical methods or officially announced methods.

EXAMPLES

The present invention and the disclosures herein will be described below by way of Examples. The present invention should not be construed as being limited to these Examples.

<Production of testing device>

A testing device was produced in the manner described below.

-Gene recombinant yeast-

For producing a recombinant, a budding yeast YIL015W BY4741 (available from ATCC, ATCC4001408) was used as a carrier cell for one copy of a specific nucleic acid sequence. The specific nucleic acid sequence was a dense nucleic acid sample DNA600-G (available from National Institute of Advanced Industrial Science and Technology, NMIJ CRM 6205-a). In the form of a plasmid produced by arranging the specific nucleic acid sequence in tandem with URA3, which was a selectable marker, one copy of the specific nucleic acid sequence was introduced into yeast genome DNA by homologous recombination, targeting a BAR1 region of the carrier cell, to produce a gene recombinant yeast.

-Culturing and cell-cycle control-

In an Erlenmeyer flask, a 90 mL fraction of the gene recombinant yeast cultured in 50 g/L of a YPD medium (available from Takara Bio Inc., CLN-630409) was mixed with 900 microliters of α1- MATING FACTOR ACETATE SALT (available from Sigma-Aldrich Co., LLC, T6901-5MG, hereinafter referred to as "a factor") prepared to 500 micrograms/mL with a Dulbecco's phosphate buffered saline (available from Thermo Fisher Scientific Inc., 14190-144, hereinafter referred to as "DPBS").

Next, the resultant was incubated with a bioshaker (available from Taitec Corporation, BR-23FH) at a shaking speed of 250 rpm at a temperature of 28 degrees C for 2 hours, to synchronize the yeast at a G0/G1 phase, to obtain a yeast suspension.

- Immobilization-

Forty-five milliliters of the synchronization-confirmed yeast suspension was transferred to a centrifuge tube (available from As One Corporation, VIO-50R) and centrifuged with a centrifugal separator (available from Hitachi, Ltd., F16RN,) at a rotation speed of 3,000 rpm for 5 minutes, with subsequent supernatant removal, to obtain yeast pellets. Four milliliters of formalin (available from Wako Pure Chemical Industries, Ltd., 062-01661) was added to the obtained yeast pellets, and the resultant was left to stand still for 5 minutes, then centrifuged with subsequent supernatant removal, and suspended with addition of 10 mL of ethanol, to obtain an immobilized yeast suspension.

-Nuclear staining-

Two hundred microliters of the immobilized yeast suspension was fractionated, washed with DPBS once, and resuspended in 480 microliters of DPBS.

Next, to the resultant, 20 microliters of 20 mg/ml RNase A (available from Nippon Gene Co., Ltd., 318-06391) was added, followed by incubation with a bioshaker at 37 degrees C for 2 hours.

Next, to the resultant, 25 microliters of 20 mg/ml proteinase K (available from Takara Bio Inc., TKR-9034) was added, followed by incubation with PETIT COOL (available from Waken B Tech Co., Ltd., PETIT COOL MINIT-C) at 50 degrees C for 2 hours.

Finally, to the resultant, 6 microliters of 5 mM SYTOX GREEN NUCLEIC ACID STAIN (available from Thermo Fisher Scientific Inc., S7020) was added, followed by staining in a light-shielded environment for 30 minutes.

- Dispersing-

The stained yeast suspension was subjected to dispersion treatment using an ultrasonic homogenizer (available from Yamato Scientific Co., Ltd., LUH150,) at a power output of 30% for 10 seconds, to obtain a yeast suspension ink.

-Dispensing and cell counting-

A plate with known cell numbers was produced by counting the number of yeast cells in liquid droplets in the manner described below. Specifically, with the use of the liquid droplet forming device illustrated in FIG. 22, the yeast suspension ink was sequentially discharged into each well of a 96 plate (product name: MICROAMP 96-WELL REACTION PLATE, available from Thermo Fisher Scientific Inc.), using a piezoelectricity applying-type discharging head (available in-house) as a liquid droplet discharging unit at 10 Hz.

An image of yeast cells in a liquid droplet discharged was captured using a high-sensitivity camera (available from Tokyo Instruments Inc., SCMOS PCO.EDGE) as a light receiving unit and using a YAG laser (available from Spectra-Physics, Inc., EXPLORER ONE-532-200-KE) as a light source, and the cell number was counted by image processing with image processing software IMAGE J serving as a particle number counting unit for the captured image. In this way, a plate with known cell numbers of from 1 through 4 was produced.

-Extraction of nucleic acids-

With a Tris-EDTA (TE) buffer and ColE1 DNA (available from Wako Pure Chemical Industries, Ltd., 312-00434), ColE1/TE was prepared at 5 ng/microliter. With ColE1/TE, a Zymolyase solution of Zymolyase[R] 100T (available from Nacalai Tesque Inc., 07665-55) was prepared at 1 mg/mL.

Four microliters of the Zymolyase solution was added into each well of the produced plate with known cell numbers, incubated at 37.2 degrees C for 30 minutes, to dissolve cell walls (extraction of nucleic acids), and then thermally treated at 95 degrees C for 2 minutes, to produce the well plate.

Using the produced well plate, the detection probability for each cell number was calculated. For each of the cell numbers of from 1 through 4, twenty-one samples were subjected to real-time PCR amplification and detection. For real-time PCR measurement, first, 1 microliter of MASTER MIX (available from Thermo Fischer Scientific Inc., TAQMAN UNIVERSAL PCR MASTER MIX), 0.5 nmol of each of a forward primer and a reverse primer for amplifying the specific DNA sequence, and 0.4 nmol of a probe were added to the samples of the well plates. Subsequently, with a real-time PCR device (available from Thermo Fischer Scientific Inc., QUANTSTUDIO 7FLEX), amplification and detection were performed.

Detection number at which presence of the cell number was able to be confirmed with real-time PCR and the detection probability are presented in Table 1.

Table 1

| Cell number | Detection number | Detection probability (%) | Ct | |
|---|---|---|---|---|
| | | | Average | SD |
| 1 | 18/21 | 85.7 | 39.77 | 1.92 |
| 2 | 20/21 | 95.2 | 38.40 | 0.89 |
| 3 | 21/21 | 100 | 38.16 | 0.93 |
| 4 | 21/21 | 100 | 37.33 | 0.81 |

<Identification of detection accuracy ability for detecting testing target in sample>

The detection probability result is a pure evaluation of only the ability of the device and the reagent. Actually, the result contains an error that occurred during sampling of the sample.

The error that occurred during sampling of the sample is considered to conform to a random distribution. Therefore, the error is expressed by a Poisson distribution when the sample contains a specific number $\lambda$ of molecules (number of molecules/sample).

Accordingly, a non-detection probability E(k) with respect to a number k of molecules can be obtained by an experiment using the standard substance of the present disclosure. Here, k is a value such as 0, 1, 2, 3, ....

Next, a copy number k of DNA contained in the sample sampled in a sample amount from a population having a specific concentration $\lambda$ (number of molecules/sample) is expressed by a Poisson distribution P(k, $\lambda$) represented by a mathematical formula (1) below.

$$P(k, \lambda) = \frac{\lambda^k e^{-\lambda}}{k!}$$

$\cdots$ Mathematical formula (1)

Hence, when a convolution integral of E(k) with the sampling variation P(k, $\lambda$) is obtained according to a mathematical formula (2) below, a non-detection accuracy ability D($\lambda$) at the specific concentration $\lambda$ can be obtained.

$$D(\lambda) = \sum_{K=0}^{\infty} E(k) \cdot P(k, \lambda)$$

$\cdots$ Mathematical formula (2)

Actually, there is no case where calculation is performed from k=0 through k=infinite. In most cases, P(k, $\lambda$) becomes zero when k=5 or greater. Therefore, calculation is needed from k=0 through about k=5.

In the way described above, the detection accuracy ability for detecting the testing target nucleic acid in the sample in the testing device was identified, using the detection probability information in Table 1 and a Poisson distribution. The results are presented in FIG. 4.

From the results of FIG. 4, it can be understood that the number of nucleic acid molecules needed to ensure a detection accuracy ability of 95% or higher is 3.5 molecules. That is, when a detection result indicates a number of nucleic acid molecules of 3.5 molecules or greater, the detection result can be highly reliably identified as having a detection accuracy ability of 95%.

**Claims**

1. A detection accuracy identifying method for identifying a detection accuracy for detecting a testing target nucleic acid, the detection accuracy identifying method comprising
identifying a detection accuracy ability of the testing target nucleic acid,
using a standard substance containing a known number of nucleic acid molecules and based on a probability at which the known number of nucleic acid molecules is detected,
wherein the known number of nucleic acid molecules comprises two or more different integers.

2. The detection accuracy identifying method according to claim 1,
wherein the identifying comprises identifying the detection accuracy ability of the testing target nucleic acid by convolving the probability at which the known number of nucleic acid molecules is detected and a probability distribution $P(k, \lambda)$ of the known number of nucleic acid molecules contained in sample sampled from a sample having a specific concentration ($\lambda$) of nucleic acid molecules.

3. The detection accuracy identifying method according to claim 2,
wherein the probability distribution is a Poisson distribution.

4. The detection accuracy identifying method according to any one of claims 1 to 3,
wherein the known number of nucleic acid molecule is 10 or less.

5. The detection accuracy identifying method according to any one of claims 1 to 4,
wherein the known number of nucleic acid molecule is introduced into a nucleic acid in a nucleus of a cell.

6. The detection accuracy identifying method according to claim 5,
wherein the cell is a yeast cell.

7. A detection accuracy identifying device configured to identify a detection accuracy for detecting a testing target nucleic acid, the detection accuracy identifying device comprising
a detection accuracy ability identifying unit configured to identify a detection accuracy ability of the testing target nucleic acid, using a standard substance containing a known number of nucleic acid molecules and based on a probability at which the known number of nucleic acid molecules is detected, wherein the known number of nucleic acid molecules comprises two or more different integers.

8. A computer-readable storage medium comprising computer executable instructions that when executed by the computer will cause the computer to carry out a method according to any one of claims 1 to 6.

9. A detection accuracy identifying program for identifying a detection accuracy for detecting a testing target nucleic acid, the detection accuracy identifying program causing a computer to execute a process comprising:
identifying a detection accuracy ability of the testing target nucleic acid, using a standard substance comprising a known number of nucleic acid molecules and based on a probability at which the known number of nucleic acid molecules is detected, wherein the known number of nucleic acid molecules comprises two or more different integers.

**Patentansprüche**

1. Detektionsgenauigkeits-Identifizierungsverfahren zum Identifizieren einer Detektionsgenauigkeit zum Detektieren einer Testziel-Nukleinsäure, wobei das Detektionsgenauigkeits-Identifizierungsverfahren Folgendes umfasst:

Identifizieren einer Detektionsgenauigkeits-Fähigkeit der Testziel-Nukleinsäure,
Verwenden einer Standardsubstanz, welche eine bekannte Anzahl an Nukleinsäuremolekülen enthält und basierend auf einer Wahrscheinlichkeit, mit welcher die bekannte Anzahl an Nukleinsäuremolekülen detektiert wird, wobei die bekannte Anzahl an Nukleinsäuremolekülen zwei oder mehr unterschiedliche Ganzzahlen umfasst.

2. Detektionsgenauigkeits-Identifizierungsverfahren nach Anspruch 1,
wobei das Identifizieren Identifizieren der Detektionsgenauigkeits-Fähigkeit der Testziel-Nukleinsäure durch Konvolvieren der Wahrscheinlichkeit umfasst, mit welcher die bekannte Anzahl an Nukleinsäuremolekülen detektiert wird und einer Wahrscheinlichkeitsverteilung $P(k, \lambda)$ der bekannten Anzahl an Nukleinsäuremolekülen, welche in einer Probe enthalten ist, welche aus einer Probe mit einer spezifischen Konzentration (λ) an Nukleinsäuremolekülen entnommen wird.

3. Detektionsgenauigkeits-Identifizierungsverfahren nach Anspruch 2, wobei die Wahrscheinlichkeitsverteilung eine Poisson-Verteilung ist.

4. Detektionsgenauigkeits-Identifizierungsverfahren nach einem der Ansprüche 1 bis 3, wobei die bekannte Anzahl an Nukleinsäuremolekülen 10 oder darunter beträgt.

5. Detektionsgenauigkeits-Identifizierungsverfahren nach einem der Ansprüche 1 bis 4, wobei die bekannte Anzahl an Nukleinsäuremolekülen in eine Nukleinsäure in einen Kern einer Zelle eingebracht wird.

6. Detektionsgenauigkeits-Identifizierungsverfahren nach Anspruch 5, wobei die Zelle eine Hefezelle ist.

7. Detektionsgenauigkeits-Identifizierungsvorrichtung zum Identifizieren einer Detektionsgenauigkeit zum Detektieren einer Testziel-Nukleinsäure, wobei die Detektionsgenauigkeits-Identifizierungsvorrichtung Folgendes umfasst: eine Einheit zur Detektionsgenauigkeits-Fähigkeits-Identifizierungseinheit, welche konfiguriert ist, um eine Detektionsgenauigkeits-Fähigkeit der Testziel-Nukleinsäure zu identifizieren, unter Verwendung einer Standardsubstanz, welche eine bekannte Anzahl an Nukleinsäuremolekülen enthält und basierend auf einer Wahrscheinlichkeit, mit welcher die bekannte Anzahl an Nukleinsäuremolekülen detektiert wird, wobei die bekannte Anzahl an Nukleinsäuremolekülen zwei oder mehr unterschiedliche Ganzzahlen umfasst.

8. Computerlesbares Speichermedium, welches computerausführbare Anweisungen umfasst, welche, wenn sie durch den Computer ausgeführt werden, den Computer dazu veranlassen, ein Verfahren nach einem der Ansprüche 1 bis 6 auszuführen.

9. Detektionsgenauigkeits-Identifizierungsprogramm zum Identifizieren einer Detektionsgenauigkeit zum Detektieren einer Testziel-Nukleinsäure, wobei das Detektionsgenauigkeits-Identifizierungsprogramm einen Computer dazu veranlasst, ein Verfahren auszuführen, welches Folgendes umfasst: Identifizieren einer Detektionsgenauigkeits-Fähigkeit der Testziel-Nukleinsäure, unter Verwendung einer Standardsubstanz, welche eine bekannte Anzahl an Nukleinsäuremolekülen umfasst und basierend auf einer Wahrscheinlichkeit, mit welcher die bekannte Anzahl an Nukleinsäuremolekülen detektiert wird, wobei die bekannte Anzahl an Nukleinsäuremolekülen zwei oder mehr unterschiedliche Ganzzahlen umfasst.

**Revendications**

1. Procédé d'identification de précision de détection pour identifier une précision de détection pour détecter un acide nucléique cible sous test, le procédé d'identification de précision de détection comprenant :

l'identification d'une capacité de précision de détection de l'acide nucléique cible sous test ; et
l'utilisation d'une substance standard qui contient un nombre connu de molécules d'acide nucléique et sur la base d'une probabilité selon laquelle le nombre connu de molécules d'acide nucléique sont détectées ;
dans lequel le nombre connu de molécules d'acide nucléique comprend deux entiers différents ou plus.

2. Procédé d'identification de précision de détection selon la revendication 1,
dans lequel l'identification comprend l'identification de la capacité de précision de détection de l'acide nucléique cible sous test en réalisant la convolution de la probabilité selon laquelle le nombre connu de molécules d'acide nucléique sont détectées et d'une distribution de probabilité $P(k, \lambda)$ du nombre connu de molécules d'acide nucléique qui sont contenues dans un échantillon qui est échantillonné à partir d'un échantillon qui présente une concentration spécifique (λ) de molécules d'acide nucléique.

3. Procédé d'identification de précision de détection selon la revendication 2, dans lequel la distribution de probabilité est une distribution de Poisson.

4. Procédé d'identification de précision de détection selon l'une quelconque des revendications 1 à 3, dans lequel le nombre connu de molécules d'acide nucléique est de 10 ou moins.

**5.** Procédé d'identification de précision de détection selon l'une quelconque des revendications 1 à 4, dans lequel le nombre connu de molécules d'acide nucléique sont introduites à l'intérieur d'un acide nucléique dans un noyau d'une cellule.

**6.** Procédé d'identification de précision de détection selon la revendication 5, dans lequel la cellule est une cellule de levure.

**7.** Dispositif d'identification de précision de détection configuré pour identifier une précision de détection pour détecter un acide nucléique cible sous test, le dispositif d'identification de précision de détection comprenant :
une unité d'identification de capacité de précision de détection qui est configurée pour identifier une capacité de précision de détection de l'acide nucléique cible sous test, en utilisant une substance standard qui contient un nombre connu de molécules d'acide nucléique et sur la base d'une probabilité selon laquelle le nombre connu de molécules d'acide nucléique sont détectées, dans lequel le nombre connu de molécules d'acide nucléique comprend deux entiers différents ou plus.

**8.** Support de stockage pouvant être lu par un ordinateur et comprenant des instructions pouvant être exécutées par un ordinateur qui, lorsqu'elles sont exécutées par l'ordinateur, forcent l'ordinateur à mettre en œuvre un procédé selon l'une quelconque des revendications 1 à 6.

**9.** Programme d'identification de précision de détection pour identifier une précision de détection pour détecter un acide nucléique cible sous test, le programme d'identification de précision de détection amenant un ordinateur à exécuter un processus comprenant :
l'identification d'une capacité de précision de détection de l'acide nucléique cible sous test, en utilisant une substance standard qui contient un nombre connu de molécules d'acide nucléique et sur la base d'une probabilité selon laquelle le nombre connu de molécules d'acide nucléique sont détectées, dans lequel le nombre connu de molécules d'acide nucléique comprend deux entiers différents ou plus.

## FIG. 1A

| Cell number | Detection number | Detection probability (%) | Ct | |
|---|---|---|---|---|
| | | | Ave. | SD |
| 1 | 18/21 | 85.7 | 39.77 | 1.92 |
| 2 | 20/21 | 95.2 | 38.40 | 0.89 |
| 3 | 21/21 | 100 | 38.16 | 0.93 |
| 4 | 21/21 | 100 | 37.33 | 0.81 |

## FIG. 1B

FIG. 2

# FIG. 3

# FIG. 4

# FIG. 5

~100

**Detection accuracy identifying device**

101 CPU

102 Main memory device

103 Auxiliary memory device

104 Output device

105 Input device

106 Communication interface

107

# FIG. 6

~100

**Detection accuracy identifying device**

~130 Control unit

~110 Input unit

~120 Output unit

~131 Detection probability information obtaining unit

~132 Probability distribution obtaining unit

~133 Detection accuracy ability identifying unit

~140 Memory unit

141 Detection probability information DB

142 Probability distribution DB

143 Detection accuracy ability DB

# FIG. 7

```
              ┌──────────┐
              │  Start   │
              └──────────┘
                    │
                    ▼           ┌S110
   ┌──────────────────────────────────────────┐
   │  Detection probability information is obtained │
   └──────────────────────────────────────────┘
                    │
                    ▼           ┌S111
   ┌──────────────────────────────────────────┐
   │      Probability distribution is obtained      │
   └──────────────────────────────────────────┘
                    │
                    ▼           ┌S112
   ┌──────────────────────────────────────────┐
   │      Detection accuracy ability is identified     │
   └──────────────────────────────────────────┘
                    │
                    ▼           ┌S113
   ┌──────────────────────────────────────────┐
   │       Detection accuracy ability is stored       │
   └──────────────────────────────────────────┘
                    │
                    ▼
              ┌──────────┐
              │   End    │
              └──────────┘
```

# FIG. 8

# FIG. 9

# FIG. 10

Wells filled with known
numbers of nucleic
acid molecule

Wells filled with sample

| 1 | 1 | | | | | | |
|---|---|---|---|---|---|---|---|
| 2 | 2 | | | | | | |
| 3 | 3 | | | | | | |
| 4 | 4 | | | | | | |
| 5 | 5 | | | | | | |

# FIG. 11

Wells filled with known
numbers of nucleic
acid molecule

Wells filled with sample

| 1 | 1 | | | | | | |
|---|---|---|---|---|---|---|---|
| 3 | 3 | | | | | | |
| 5 | 5 | | | | | | |
| 7 | 7 | | | | | | |
| 9 | 9 | | | | | | |

# FIG. 12

Fluorescence intensity

# FIG. 13A

# FIG. 13B

# FIG. 13C

# FIG. 14A

# FIG. 14B

## FIG. 15A

11c

12c

13c

## FIG. 15B

11c

12c

13c

## FIG. 15C

11c

12c

13c

310'

# FIG. 16

# FIG. 17

# FIG. 18

Control unit ⌒70

| CPU ⌒71 | ROM ⌒72 | RAM ⌒73 |

I/F ⌒74

75

# FIG. 19

Control unit ⌒70

Discharging control unit ⌒701

Light source control unit ⌒702

Cell number counting unit ⌒703

# FIG. 20

```
        ( Start )
            |
            |              S11
+---------------------------+
| Liquid droplet is discharged |
+---------------------------+
            |
            |              S12
+---------------------------+
|  Light source is turned on  |
+---------------------------+
            |
            |              S13
+---------------------------+
|   Cell number is counted    |
+---------------------------+
            |
            |
        ( End )
```

# FIG. 21

# FIG. 22

# FIG. 23A

350₁    350₂

# FIG. 23B

350₁    350₂

# FIG. 24

# FIG. 25

# FIG. 26

# FIG. 27

# FIG. 28

# FIG. 29

## REFERENCES CITED IN THE DESCRIPTION

**Patent documents cited in the description**

- JP 2014033658 A
- JP 2015195735 A

**Non-patent literature cited in the description**

- *Chronicles of Young Scientists,* January 2011, vol. 2, cysonline.org.on
- *Journal of AOAC International,* vol. 94 (1), 335-347
- **FOROOTAN et al.** Methods to determine limit of detection and limit of quantification in quantitative real-time PCR (qPCR). *Biomolecular Detection and Quantification,* 01 June 2017, vol. 12, 1-6
- **MANO et al.** Development of a reference material of a single DNA molecule for the quality control of PCR testing. *Analytical Chenistry,* 12 August 2014, vol. 86 (17), 8621-8627
- **SANGJUN et al.** *PLoS One,* vol. 6 (3), e17455